# EUROPEAN PATENT APPLICATION

(11) **EP 3 395 165 A1**
(43) Date of publication of application: **31.10.2018**
(21) Application number: 18176859.9
(22) Date of filing: 04.10.2013
(51) Int. Cl.: A01H 1/00, A01H 5/00, A01P 7/00, C12N 15/82, A01N 63/02

(54) **USE OF CRY1EA IN COMBINATIONS FOR MANAGEMENT OF RESISTANT FALL ARMYWORM INSECTS**

(30) Priority: 05.10.2012 US 201261710154 P
(62) Divisional of application: 13844137.3
(71) Applicant: Dow AgroSciences LLC, Indianapolis, IN 46268 (US)
(72) Inventor: SHEETS, Joel. J, San Luis Obispo, CA 93401-4648 (US); NARVA, Kenneth. E, Zionsville, IN 46077 (US); BURTON, Stephanie, Indianapolis, IN 46278 (US); CALDWELL, Elizabeth, A, Indianapolis, IN 46268 (US)
(74) Representative: HGF Limited

(57) **Abstract**

The subject invention includes methods and plants for controlling fall armyworm lepidopteran insects, said plants comprising a CrylEa insecticidal protein and a second insecticidal protein selected from the group of CrylAb, CrylBe, CrylCa, CrylDa, and Vip3Ab to delay or prevent development of resistance by the insect

## Description

### CROSS REFERENCE

This application claims the benefit of US provisional application 61/710,154, filed on October 5, 2012. The entire disclosure of which is expressly incorporated herein by reference.

### BACKGROUND OF THE INVENTION

Humans grow corn for food and energy applications. Humans also grow many other crops, including soybeans and cotton. Insects eat and damage plants and thereby undermine these human efforts. Billions of dollars are spent each year to control insect pests and additional billions are lost to the damage they inflict. Synthetic organic chemical insecticides have been the primary tools used to control insect pests but biological insecticides, such as the insecticidal proteins derived from *Bacillus thuringiensis* (*Bt*), have played an important role in some areas. The ability to produce insect-resistant plants through transformation with *Bt* insecticidal protein genes has revolutionized modern agriculture and heightened the importance and value of insecticidal proteins and their genes.

Several *Bt* proteins have been used to create the insect-resistant transgenic plants that have been successfully registered and commercialized to date. These include Cry1Ab, Cry1Ac, Cry1F and Cry3Bb in corn, Cry1Ac and Cry2Ab in cotton, and Cry3A in potato.

The commercial products expressing these proteins express a single protein except in cases where the combined insecticidal spectrum of 2 proteins is desired (*e.g*., Cry1Ab and Cry3Bb in corn combined to provide resistance to lepidopteran pests and rootworm, respectively) or where the independent action of the proteins makes them useful as a tool for delaying the development of resistance in susceptible insect populations (*e.g*., Cry1Ac and Cry2Ab in cotton combined to provide resistance management for tobacco budworm). *See also* U.S. Patent Application Publication No. 2009/0313717, which relates to a Cry2 protein plus a Vip3Aa, Cry1F, or Cry1A for control of *Helicoverpa zea* or *armigerain.* WO 2009/132850 relates to Cry1F or Cry1A and Vip3Aa for controlling *Spodoptera frugiperda.* U.S. Patent Application Publication No. 2008/0311096 relates in part to Cry1Ab for controlling Cry1F-resistant ECB.

That is, some of the qualities of insect-resistant transgenic plants that have led to rapid and widespread adoption of this technology also give rise to the concern that pest populations will develop resistance to the insecticidal proteins produced by these plants. Several strategies have been suggested for preserving the utility of *Bt*-based insect resistance traits which include deploying proteins at a high dose in combination with a refuge, and alternation with, or co-deployment of, different toxins (McGaughey et al. (1998), "B.t. Resistance Management," Nature Biotechnol. 16:144-146).

The protein toxins selected for use in an insect resistant management (IRM) stack need to exert their insecticidal effect independently so that resistance developed to one protein does not confer resistance to the second protein (*i.e.*, there is no cross resistance to the proteins). If, for example, a pest population that is resistant to "Protein A" is sensitive to "Protein B", one would conclude that there is no cross resistance and that a combination of Protein A and Protein B would be effective in delaying resistance to Protein A alone.

In the absence of resistant insect populations, assessments can be made based on other characteristics presumed to be related to mechanism of action and cross-resistance potential. The utility of receptor-mediated binding in identifying insecticidal proteins likely to not exhibit cross resistance has been suggested (van Mellaert *et al.* 1999). The key predictor of lack of cross resistance inherent in this approach is that the insecticidal proteins do not compete for receptors in a sensitive insect species.

In the event that two *Bt* toxins compete for the same receptor, then if that receptor mutates in that insect so that one of the toxins no longer binds to that receptor and thus is no longer insecticidal against the insect, it might be the case that the insect will also be resistant to the second toxin (which competitively bound to the same receptor). That is, the insect is said to be cross-resistant to both *Bt* toxins. However, if two toxins bind to two different receptors, this could be an indication that the insect would not simultaneously develop resistance to those two toxins.

Representative *Cry* toxins are listed at the website of the official *Bt* nomenclature committee (Crickmore *et al*.; lifesci.sussex.ac.uk/home/Neil_Crickmore/Bt/). There are currently nearly 60 main groups of "Cry" proteins (Cry1-Cry59), with additional Cyt proteins and VIP proteins and the like. Many of each numeric group have capital-letter subgroups, and the capital letter subgroups have lower-cased letter sub-subgroups. (Cry1 has A-L, and Cry1A has a-i, for example).

### BRIEF SUMMARY OF THE INVENTION

The subject invention relates in part to the surprising discovery that the insecticidal protein, Cry1Ea, does not compete with Cry1Ab, Cry1Be, Cry1Ca, Cry1Da, or VIP3Ab for binding with fall armyworm (FAW; *Spodoptera frugiperda*) gut cell membrane receptor preparations. As one skilled in the art will recognize with the benefit of this disclosure, plants that produce any of the subject pairs of proteins (including insecticidal portions of the full-length proteins), which do not competitively bind with each other, can delay or prevent the development of resistance to any of these insecticidal proteins alone.

Thus, the subject invention relates in part to the use of a Cry1Ea protein in combination with a Cry1Ab, Cry1Be, Cry1Ca, Cry1Da, and/or a VIP3Ab protein. Plants (and acreage planted with such plants) that produce CrylEa in combination with at least one of the other proteins described herein are included within the scope of the subject invention.

The subject invention also relates in part to triple stacks or "pyramids" of three (or more) toxins, with Cry1Ea being at least one of the proteins in the stack. In some preferred pyramid embodiments, the combination of the selected toxins provides non-cross-resistant action against FAW. Some preferred "three sites of action" pyramid combinations include one of the subject base pair of proteins (Cry1Ea plus Cry1Ab, Cry1Ca, Cry1Da, Vip3Ab, or Cry1Be) and an additional *Bt* protein for targeting FAW. These particular triple stacks would, according to the subject invention, advantageously and surprisingly provide three sites of action against FAW. This can help to reduce or eliminate the requirement for refuge acreage.

### BRIEF DESCRIPTION OF THE FIGURES

FIG. 1 shows percent specific binding of ¹²⁵I Cry1Ea (0.5 nM) in BBMV's from FAW versus competition by unlabeled homologous CrylEa and heterologous Cry1Ab.
FIG. 2 shows the binding of ¹²⁵I Cry1Ab to BBMV's from FAW larvae and its subsequent displacement by increasing concentrations of unlabeled Cry1Ab.
FIG. 3 shows percent specific binding of ¹²⁵I CryvEa in BBMV's from FAW versus competition by unlabeled homologous Cry1Ea and heterologous Cry1Ca.
FIG. 4 shows the results of a binding assay in which ¹²⁵I Cry1Ea was bound to BBMV's from FAW larvae and Cry1Ea, Cry1Be, Cry1Da, and VIP3Ab1 ligands were subsequently added.

### BRIEF DESCRIPTION OF THE SEQUENCES

SEQ ID NO: 1 shows the amino acid sequence of an 1148-amino-acid Cry1Ea protein (with a CryvAb protoxin segment).
SEQ ID NO: 2 shows the amino acid sequence of a full-length (1155 amino acids) CrylAb protein.
SEQ ID NO: 3 shows the amino acid sequence of an 1186-amino-acid Cry1Be protein (with a Cry1Ab protoxin segment).
SEQ ID NO: 4 shows the amino acid sequence of an 1164-amino-acid Cry1Ca protein (with a Cry1Ab protoxin segment).
SEQ ID NO: 5 shows the amino acid sequence of an 1139-amino-acid Cry1Da protein (with a Cry1Ab protoxin segment).
SEQ ID NO: 6 shows the amino acid sequence of a full-length Vip3Ab protein.
SEQ ID NO: 7 shows the amino acid sequence of a protease-processed Cry1Ea protein.
SEQ ID NO: 8 shows the amino acid sequence of a protease-processed Cry1Ab protein.
SEQ ID NO: 9 shows the amino acid sequence of a protease-processed Cry1Be protein.
SEQ ID NO: 10 shows the amino acid sequence of a protease-processed Cry1Ca protein.
SEQ ID NO: 11 shows the amino acid sequence of a protease-processed Cry1Da protein.

### DETAILED DESCRIPTION OF THE INVENTION

The subject invention relates in part to the surprising discovery that Cry1Ea has a unique and independent mode of action and does not compete with Cry1Ab, Cry1Be, Cry1Ca, Cry1Da, or VIP3Ab1 for binding sites in the gut of fall armyworms (FAW; *Spodoptera frugiperda).* Thus, a Cry1Ea protein can be used in combination with Cry1Ab, Cry1Be, Cry1Ca, Cry1Da, and/or VIP3Ab1 proteins in transgenic corn (and other plants; e.g., cotton, for example) to delay or prevent FAW from developing resistance to these proteins alone. The subject protein, used together with the other Cry or VIP proteins disclosed herein, can be effective at protecting plants (such as maize plants and/or soybean plants) from damage by Cry-resistant fall armyworm. That is, one use of the subject invention is to protect corn and other economically important plant species from damage and yield loss caused by fall armyworm populations that could develop resistance to a single Cry or VIP protein.

The subject invention thus teaches an insect resistant management (IRM) stack comprising Cry1Ea and Cry1Ab, Cry1Be, Cry1Ca, Cry1Da, and/or VIP3Ab1 to prevent or mitigate the development of resistance by FAW to one of these proteins used by itself.

The present invention includes compositions for controlling FAW, wherein the compositions comprise a CrylEa insecticidal protein and a Cry1Ab, Cry1Be, Cry1Ca, Cry1Da, and/or VIP3Ab1 insecticidal protein. The subject compositions include plants and plant cells.

The invention further comprises a host transformed to produce both a CryEa insecticidal protein and a Cry1Ab, Cry1Be, Cry1Ca, Cry1Da, or VIP3Ab1 insecticidal protein, wherein said host is a microorganism or a plant cell. The subject polynucleotide(s) are preferably in a genetic construct under control of a non-*Bacillus-thuringiensis* promoter(s). The terms "isolated" and "heterologous" connote that the polypeptide or DNA molecules are in a state that is different from their native environment - involving the hand of man. The subject polynucleotides can comprise codon usage for enhanced expression in a plant.

In addition, the invention includes a method of controlling FAWs, wherein the method comprises contacting said FAW or the environment of said FAW with an effective amount of a composition that contains a Cry1Ea core toxin-containing protein pair of the subject invention.

An embodiment of the invention comprises a plant (which can include corn, soybeans, and cotton) comprising plant-expressible genes encoding a Cry1Ea insecticidal protein pair of the subject invention, and seed of such a plant.

A further embodiment of the invention comprises a plant wherein plant-expressible genes encoding a Cry1Ea insecticidal protein pair of the subject invention have been introgressed into said plant, and seed of such a plant.

As described in the Examples, competitive receptor binding studies using radiolabeled Cry1Ea protein and/or radiolabled Cry1Ab protein show that the Cry1Ab, Cry1Be, Cry1Ca, Cry1Da, and VIP3Ab1 proteins do not compete with Cry1Ea for binding in FAW midgut brush border membrane vesicles to which Cry1Ea binds. These results also indicate that the combination of Cry1Ea and these other proteins can be an effective means to mitigate the development of resistance in FAW populations to these proteins. Thus, based in part on the data described herein, it is thought that co-production (stacking) of the Cry1Ea and Cry1Ab, Cry1Ca, Cry1Be, Cry1Da, and/or VIP3Ab1 proteins can be used to produce a high dose IRM stack for controlling/inhibiting/killing FAW.

Other proteins can be added to a subject pair. For example, the subject invention also relates in part to triple stacks or "pyramids" of three (or more) toxins, with a subject pair being the base pair. In some preferred pyramid embodiments, the selected toxins have three separate modes of action against FAW. Some preferred "three modes of action" pyramid combinations include the subject base pair of proteins plus a third protein for targetting FAW. By "separate sites of action," it is meant any of the given proteins do not cause cross-resistance with each other. These particular triple stacks would, according to the subject invention, advantageously and surprisingly provide three sites of action against FAW. This can help to reduce or eliminate the requirement for refuge acreage.

Thus, one deployment option is to use the subject pair of proteins in combination with a third toxin/gene, and to use this triple stack to mitigate the development of resistance in FAW to any of these toxins. Accordingly, the subject invention also relates in part to triple stacks or "pyramids" of three (or more) toxins. In some preferred pyramid embodiments, the selected toxins have three separate sites of action against FAW.

Included among deployment options of the subject invention would be to use two, three, or more proteins of the subject proteins in crop-growing regions where FAW can develop resistant populations.

Plants (and acreage planted with such plants) that produce any of the subject combinations of proteins are included within the scope of the subject invention. Additional toxins/genes can also be added, but the particular stacks discussed above advantageously and surprisingly provide multiple sites of action against FAW. This can help to reduce or eliminate the requirement for refuge acreage. A field thus planted of over ten acres is thus included within the subject invention.

GENBANK can also be used to obtain the sequences for any of the genes and proteins disclosed or mentioned herein. Relevant sequences are also available in patents. Representative *Cry* toxins are listed at the website of the official *Bt* nomenclature committee (Crickmore *et al*.; lifesci.sussex.ac.uk/home/Neil_Crickmore/Bt/). There are currently about 70 main groups of "Cry" proteins (Cry1-Cry70), with additional VIP proteins and the like.

Combinations of proteins described herein can be used to control lepidopteran pests. Adult lepidopterans, for example, butterflies and moths, primarily feed on flower nectar and are a significant effector of pollination. Nearly all lepidopteran larvae, *i.e*., caterpillars, feed on plants, and many are serious pests. Caterpillars feed on or inside foliage or on the roots or stem of a plant, depriving the plant of nutrients and often destroying the plant's physical support structure. Additionally, caterpillars feed on fruit, fabrics, and stored grains and flours, ruining these products for sale or severely diminishing their value. As used herein, reference to lepidopteran pests refers to various life stages of the pest, including larval stages.

Some chimeric proteins of the subject invention comprise a full N-terminal core toxin region of a full *Bt* protein toxin and, at some point past the end of the core toxin portion, the protein has a transition to a heterologous protoxin sequence. The N-terminal, insecticidally active, toxin portion of a *Bt* protein toxin is referred to as the "core" toxin. The portion that is C-terminal to the core toxin is referred to as the "protoxin" segment or portion. The transition from the core toxin segment to the heterologous protoxin segment can occur at approximately the core toxin/protoxin junction or, in the alternative, a portion of the native protoxin can be retained, with the transition to the heterologous protoxin portion occurring downstream.

As an example, one chimeric protein of the subject invention, is a full core toxin portion of CryvEa (roughly the first 600 amino acids) and/or a heterologous protoxin (the remaining amino acids to the C-terminus). In one preferred embodiment, the portion of a chimeric protein comprising the protoxin is derived from a Cry1Ab toxin. Aside from Vip3Ab, all of the proteins for use according to the subject invention share similar full-length and core toxin sizes and structures.

A person skilled in this art will appreciate that *Bt* protein toxins, even within a certain class such as Cry1Ea, will vary to some extent in length, and the precise location of the transition from core toxin to protoxin will also vary. Typically, the Cry1Ea proteins, for example, are about 1150 to about 1200 amino acids in length. The transition from core toxin portion to protoxin portion will typically occur at between about 50% to about 60% of the full length toxin. The chimeric proteins of the subject invention will include the full expanse of this N-terminal core toxin region. Thus, the chimeric protein will comprise at least about 50% of the full length of the Cry1 *Bt* toxin protein. This will typically be at least about 590 amino acids. With regard to the protoxin portion, the full expanse of the CryvAb protoxin portion extends from the end of the core toxin portion to the C-terminus of the molecule.

Genes and toxins. The genes and toxins useful according to the subject invention include not only the full length sequences disclosed but also fragments of these sequences, variants, mutants, and fusion proteins which retain the characteristic insecticidal activity of the core toxins specifically exemplified herein. As used herein, the terms "variants" or "variations" of genes refer to nucleotide sequences which encode the same toxins or which encode equivalent toxins having pesticidal activity. As used herein, the term "equivalent toxins" refers to toxins having the same or essentially the same biological activity against the target pests as the claimed toxins.

As used herein, the boundaries represent approximately 95% (Cry1Ea's), 78% (Cry1E's), and 45% (Cry1's) sequence identity, per "Revision of the Nomenclature for the Bacillus thuringiensis Pesticidal Crystal Proteins," N. Crickmore, D.R. Zeigler, J. Feitelson, E. Schnepf, J. Van Rie, D. Lereclus, J. Baum, and D.H. Dean. Microbiology and Molecular Biology Reviews (1998) Vol 62: 807-813. These cut offs can also be applied to the core toxins only.

It will be apparent to a person skilled in this art that genes encoding active toxins can be identified and obtained through several means. The specific genes or gene portions exemplified herein may be obtained from the isolates deposited at a culture depository. These genes, or portions or variants thereof, may also be constructed synthetically, for example, by use of a gene synthesizer. Variations of genes may be readily constructed using standard techniques for making point mutations. Also, fragments of these genes can be made using commercially available exonucleases or endonucleases according to standard procedures. For example, enzymes such as Bal31 or site-directed mutagenesis can be used to systematically cut off nucleotides from the ends of these genes. Genes that encode active fragments may also be obtained using a variety of restriction enzymes. Proteases may be used to directly obtain active fragments of these protein toxins.

Fragments and equivalents which retain the pesticidal activity of the exemplified toxins are within the scope of the subject invention. Also, because of the redundancy of the genetic code, a variety of different DNA sequences can encode the amino acid sequences disclosed herein. It is well within the skill of a person trained in the art to create these alternative DNA sequences encoding the same, or essentially the same, toxins. These variant DNA sequences are within the scope of the subject invention. As used herein, reference to "essentially the same" sequence refers to sequences which have amino acid substitutions, deletions, additions, or insertions which do not materially affect pesticidal activity. Fragments of genes encoding proteins that retain pesticidal activity are also included in this definition.

A further method for identifying the genes encoding the toxins and gene portions useful according to the subject invention is through the use of oligonucleotide probes. These probes are detectable nucleotide sequences. These sequences may be detectable by virtue of an appropriate label or may be made inherently fluorescent as described in International Application No. WO93/16094. As is well known in the art, if the probe molecule and nucleic acid sample hybridize by forming a strong bond between the two molecules, it can be reasonably assumed that the probe and sample have substantial homology. Preferably, hybridization is conducted under stringent conditions by techniques well-known in the art, as described, for example, in Keller, G. H., M. M. Manak (1987) DNA Probes, Stockton Press, New York, N.Y., pp. 169-170. Some examples of salt concentrations and temperature combinations are as follows (in order of increasing stringency): 2X SSPE or SSC at room temperature; 1X SSPE or SSC at 42° C; 0.1X SSPE or SSC at 42° C; 0.1X SSPE or SSC at 65° C. Detection of the probe provides a means for determining in a known manner whether hybridization has occurred. Such a probe analysis provides a rapid method for identifying toxin-encoding genes of the subject invention. The nucleotide segments which are used as probes according to the invention can be synthesized using a DNA synthesizer and standard procedures. These nucleotide sequences can also be used as PCR primers to amplify genes of the subject invention.

Variant toxins. Certain toxins of the subject invention have been specifically exemplified herein. Since these toxins are merely exemplary of the toxins of the subject invention, it should be readily apparent that the subject invention comprises variant or equivalent toxins (and nucleotide sequences coding for equivalent toxins) having the same or similar pesticidal activity of the exemplified toxin. Equivalent toxins will have amino acid homology with an exemplified toxin. This amino acid homology will typically be greater than 75%, preferably be greater than 90%, and most preferably be greater than 95%. The amino acid homology will be highest in critical regions of the toxin which account for biological activity or are involved in the determination of three-dimensional configuration which ultimately is responsible for the biological activity. In this regard, certain amino acid substitutions are acceptable and can be expected if these substitutions are in regions which are not critical to activity or are conservative amino acid substitutions which do not affect the three-dimensional configuration of the molecule. For example, amino acids may be placed in the following classes: non-polar, uncharged polar, basic, and acidic. Conservative substitutions whereby an amino acid of one class is replaced with another amino acid of the same type fall within the scope of the subject invention so long as the substitution does not materially alter the biological activity of the compound. Below is a listing of examples of amino acids belonging to each class.

| **Class of Amino Acid** | **Examples of Amino Acids** |
|---|---|
| Nonpolar | Ala, Val, Leu, Ile, Pro, Met, Phe, Trp |
| Uncharged Polar | Gly, Ser, Thr, Cys, Tyr, Asn, Gln |
| Acidic | Asp, Glu |
| Basic | Lys, Arg, His |

In some instances, non-conservative substitutions can also be made. The critical factor is that these substitutions must not significantly detract from the biological activity of the toxin.

Recombinant hosts. The genes encoding the toxins of the subject invention can be introduced into a wide variety of microbial or plant hosts. Expression of the toxin gene results, directly or indirectly, in the intracellular production and maintenance of the pesticide. Conjugal transfer and recombinant transfer can be used to create a *Bt* strain that expresses both toxins of the subject invention. Other host organisms may also be transformed with one or both of the toxin genes then used to accomplish the synergistic effect. With suitable microbial hosts, *e.g.*, *Pseudomonas*, the microbes can be applied to the situs of the pest, where they will proliferate and be ingested. The result is control of the pest. Alternatively, the microbe hosting the toxin gene can be treated under conditions that prolong the activity of the toxin and stabilize the cell. The treated cell, which retains the toxic activity, then can be applied to the environment of the target pest.

Where the *Bt* toxin gene is introduced via a suitable vector into a microbial host, and said host is applied to the environment in a living state, it is essential that certain host microbes be used. Microorganism hosts are selected which are known to occupy the "phytosphere" (phylloplane, phyllosphere, rhizosphere, and/or rhizoplane) of one or more crops of interest. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the pesticide from environmental degradation and inactivation.

A large number of microorganisms are known to inhabit the phylloplane (the surface of the plant leaves) and/or the rhizosphere (the soil surrounding plant roots) of a wide variety of important crops. These microorganisms include bacteria, algae, and fungi. Of particular interest are microorganisms, such as bacteria, *e.g.*, genera *Pseudomonas, Erwinia, Serratia, Klebsiella, Xanthomonas, Streptomyces, Rhizobium, Rhodopseudomonas, Methylophilius, Agrobactenum, Acetobacter, Lactobacillus, Arthrobacter, Azotobacter, Leuconostoc,* and *Alcaligenes*; fungi, particularly yeast, *e.g.*, genera *Saccharomyces, Cryptococcus, Kluyveromyces, Sporobolomyces, Rhodotorula,* and *Aureobasidium.* Of particular interest are such phytosphere bacterial species as *Pseudomonas syringae, Pseudomonas fluorescens, Serratia marcescens, Acetobacter xylinum, Agrobactenium tumefaciens, Rhodopseudomonas spheroides, Xanthomonas campestris, Rhizobium melioti, Alcaligenes entrophus,* and *Azotobacter vinlandii;* and phytosphere yeast species such as *Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces roseus, S. odorus, Kluyveromyces veronae,* and *Aureobasidium pollulans.* Of particular interest are the pigmented microorganisms.

A wide variety of methods are available for introducing a *Bt* gene encoding a toxin into a microorganism host under conditions which allow for stable maintenance and expression of the gene. These methods are well known to those skilled in the art and are described, for example, in U.S. Patent No. 5,135,867, which is incorporated herein by reference.

Treatment of cells. *Bacillus thuringiensis* or recombinant cells expressing the *Bt* toxins can be treated to prolong the toxin activity and stabilize the cell. The pesticide microcapsule that is formed comprises the *Bt* toxin or toxins within a cellular structure that has been stabilized and will protect the toxin when the microcapsule is applied to the environment of the target pest. Suitable host cells may include either prokaryotes or eukaryotes, normally being limited to those cells which do not produce substances toxic to higher organisms, such as mammals. However, organisms which produce substances toxic to higher organisms could be used, where the toxic substances are unstable or the level of application sufficiently low as to avoid any possibility of toxicity to a mammalian host. As hosts, of particular interest will be the prokaryotes and the lower eukaryotes, such as fungi.

The cell will usually be intact and be substantially in the proliferative form when treated, rather than in a spore form, although in some instances spores may be employed.

Treatment of the microbial cell, e.g., a microbe containing the *Bt* toxin gene or genes, can be by chemical or physical means, or by a combination of chemical and/or physical means, so long as the technique does not deleteriously affect the properties of the toxin, nor diminish the cellular capability of protecting the toxin. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as glutaraldehyde; antiinfectives, such as zephiran chloride and cetylpyridinium chloride; alcohols, such as isopropyl and ethanol; various histologic fixatives, such as Lugol iodine, Bouin's fixative, various acids and Helly's fixative (See: Humason, Gretchen L., Animal Tissue Techniques, W. H. Freeman and Company, 1967); or a combination of physical (heat) and chemical agents that preserve and prolong the activity of the toxin produced in the cell when the cell is administered to the host environment. Examples of physical means are short wavelength radiation such as gamma-radiation and X-radiation, freezing, UV irradiation, lyophilization, and the like. Methods for treatment of microbial cells are disclosed in U.S. Pat. Nos. 4,695,455 and 4,695,462, which are incorporated herein by reference.

The cells generally will have enhanced structural stability which will enhance resistance to environmental conditions. Where the pesticide is in a proform, the method of cell treatment should be selected so as not to inhibit processing of the proform to the mature form of the pesticide by the target pest pathogen. For example, formaldehyde will crosslink proteins and could inhibit processing of the proform of a polypeptide pesticide. The method of treatment should retain at least a substantial portion of the bio-availability or bioactivity of the toxin.

Characteristics of particular interest in selecting a host cell for purposes of production include ease of introducing the Bt gene or genes into the host, availability of expression systems, efficiency of expression, stability of the pesticide in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; survival in aqueous environments; lack of mammalian toxicity; attractiveness to pests for ingestion; ease of killing and fixing without damage to the toxin; and the like. Other considerations include ease of formulation and handling, economics, storage stability, and the like.

Growth of cells. The cellular host containing the *Bt* insecticidal gene or genes may be grown in any convenient nutrient medium, where the DNA construct provides a selective advantage, providing for a selective medium so that substantially all or all of the cells retain the *Bt* gene. These cells may then be harvested in accordance with conventional ways. Alternatively, the cells can be treated prior to harvesting.

The *Bt* cells producing the toxins of the invention can be cultured using standard art media and fermentation techniques. Upon completion of the fermentation cycle the bacteria can be harvested by first separating the *Bt* spores and crystals from the fermentation broth by means well known in the art. The recovered *Bt* spores and crystals can be formulated into a wettable powder, liquid concentrate, granules or other formulations by the addition of surfactants, dispersants, inert carriers, and other components to facilitate handling and application for particular target pests. These formulations and application procedures are all well known in the art.

Formulations. Formulated bait granules containing an attractant and spores, crystals, and toxins of the *Bt* isolates, or recombinant microbes comprising the genes obtainable from the *Bt* isolates disclosed herein, can be applied to the soil. Formulated product can also be applied as a seed-coating or root treatment or total plant treatment at later stages of the crop cycle. Plant and soil treatments of *Bt* cells may be employed as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

As would be appreciated by a person skilled in the art, the pesticidal concentration will vary widely depending upon the nature of the particular formulation, particularly whether it is a concentrate or to be used directly. The pesticide will be present in at least 1% by weight and may be 100% by weight. The dry formulations will have from about 1-95% by weight of the pesticide while the liquid formulations will generally be from about 1-60% by weight of the solids in the liquid phase. The formulations will generally have from about 10² to about 10⁴ cells/mg. These formulations will be administered at about 50 mg (liquid or dry) to 1 kg or more per hectare.

The formulations can be applied to the environment of the lepidopteran pest, e.g., foliage or soil, by spraying, dusting, sprinkling, or the like.

Plant transformation. Some preferred recombinant hosts in which to express the insecticidal proteins of the subject invention are plants. More highly preferred hosts are crop plants commonly used to produce food, feed, fuel, and oils. More highly preferred host crop plants are maize, soy, cotton, and canola. Maize is a highly preferred embodiment. Genes encoding *Bt* toxin proteins, as disclosed herein, can be inserted into plant cells using a variety of techniques which are well known in the art. For example, a large number of cloning vectors comprising a replication system in *Escherichia coli* and a marker that permits selection of the transformed cells are available for preparation for the insertion of foreign genes into higher plants. The vectors comprise, for example, pBR322, pUC series, M13mp series, pACYC184, *inter alia.* Accordingly, the DNA fragment having the sequence encoding the *Bt* toxin protein can be inserted into the vector at a suitable restriction site. The resulting plasmid is used for transformation into *E. coli.* The *E. coli* cells are cultivated in a suitable nutrient medium, then harvested and lysed. The plasmid is recovered. Sequence analysis, restriction analysis, electrophoresis, and other biochemical-molecular biological methods are generally carried out as methods of analysis. After each manipulation, the DNA sequence used can be cleaved and joined to the next DNA sequence. Each plasmid sequence can be cloned in the same or other plasmids. Depending on the method of inserting desired genes into the plant, other DNA sequences may be necessary. If, for example, the Ti or Ri plasmid is used for the transformation of the plant cell, then at least the right border, but often the right and the left border of the Ti or Ri plasmid T-DNA, has to be joined as the flanking region of the genes to be inserted. The use of T-DNA for the transformation of plant cells has been intensively researched and sufficiently described in EP 120 516, Lee and Gelvin (2008), Hoekema (1985), Fraley *et al*., (1986), and An *et al*., (1985), and is well established in the art.

Once the inserted DNA has been integrated in the plant genome, it is relatively stable. The transformation vector normally contains a selectable marker that confers on the transformed plant cells resistance to a biocide or an antibiotic, such as Bialaphos, Kanamycin, G418, Bleomycin, or Hygromycin, *inter alia.* The individually employed marker should accordingly permit the selection of transformed cells rather than cells that do not contain the inserted DNA.

A large number of techniques are available for inserting DNA into a plant host cell. Those techniques include transformation with T-DNA using *Agrobacterium tumefaciens* or *Agrobacterium rhizogenes* as transformation vector, fusion, injection, biolistics (microparticle bombardment), or electroporation as well as other methods including nanoparticle transformation and cell penetrating peptide mediated transformation. See for example WO 2008/148223, WO2009/046384, WO2011/046786, WO2011/126644, WO2012/006439, and WO2012/006443. If Agrobacteria are used for the transformation, the DNA to be inserted has to be cloned into special plasmids, namely either into an intermediate vector or into a binary vector. The intermediate vectors can be integrated into the Ti or Ri plasmid by homologous recombination owing to sequences that are homologous to sequences in the T-DNA. The Ti or Ri plasmid also comprises the vir region necessary for the transfer of the T-DNA. Intermediate vectors cannot replicate themselves in Agrobacteria. The intermediate vector can be transferred into *Agrobacterium tumefaciens* by means of a helper plasmid (conjugation). Binary vectors can replicate themselves both in *E. coli* and in Agrobacteria. They comprise a selection marker gene and a linker or polylinker which are framed by the Right and Left T-DNA border regions. They can be transformed directly into Agrobacteria (Holsters *et al*., 1978). The *Agrobacterium* used as host cell is to comprise a plasmid carrying a vir region. The vir region is necessary for the transfer of the T-DNA into the plant cell. Additional T-DNA may be contained. The bacterium so transformed is used for the transformation of plant cells. Plant explants can advantageously be cultivated with *Agrobacterium tumefaciens* or *Agrobacterium rhizogenes* for the transfer of the DNA into the plant cell. Whole plants can then be regenerated from the infected plant material (for example, pieces of leaf, segments of stalk, roots, but also protoplasts or suspension-cultivated cells) in a suitable medium, which may contain antibiotics or biocides for selection. The plants so obtained can then be tested for the presence of the inserted DNA. No special demands are made of the plasmids in the case of injection and electroporation. It is possible to use ordinary plasmids, such as, for example, pUC derivatives.

The transformed cells can be grown and differentiated into whole fertile plants in the usual manner. They can form germ cells and transmit the transformed trait(s) to progeny plants. Such plants can be grown in the normal manner and crossed with plants that have the same transformed hereditary factors or other hereditary factors. The resulting hybrid individuals have the corresponding phenotypic properties. Plant cells of the subject invention can also be non-totipotent / unable to be reproduced into whole plants. Such cells can include leaf cells, for example. However, the subject invention also includes cells from seeds of the subject invention, which can be reproduced into whole plants.

In a preferred embodiment of the subject invention, plants will be transformed with genes wherein the codon usage has been optimized for plants. See, for example, U.S. Patent No. 5,380,831, which is hereby incorporated by reference. While some truncated toxins are exemplified herein, it is well-known in the *Bt* art that 130 kDa-type (full-length) toxins have an N-terminal half that is the core toxin, and a C-terminal half that is the protoxin "tail." Thus, appropriate "tails" can be used with truncated / core toxins of the subject invention. *See e.g.* U.S. Patent No. 6,218,188 and U.S. Patent No. 6,673,990. In addition, methods for creating synthetic *Bt* genes for use in plants are known in the art (Stewart and Burgin, 2007). One non-limiting example of a preferred transformed plant is a fertile plant comprising a plant expressible gene encoding a Cry1Ea protein, and further comprising a second plant expressible gene encoding a Cry1Ab, Cry1Be, Cry1Ca, Cry1Da, or VIP3Ab protein.

Transfer (or introgression) of the Cry1Ea-pair traits into inbred lines can be achieved by recurrent selection breeding, for example by backcrossing. In this case, a desired recurrent parent is first crossed to a donor inbred (the non-recurrent parent) that carries the appropriate gene(s) for the Cry1Ea-pair traits. The progeny of this cross is then mated back to the recurrent parent followed by selection in the resultant progeny for the desired trait(s) to be transferred from the non-recurrent parent. After three, preferably four, more preferably five or more generations of backcrosses with the recurrent parent with selection for the desired trait(s), the progeny will be heterozygous for loci controlling the trait(s) being transferred, but will be like the recurrent parent for most or almost all other genes (see, for example, Poehlman & Sleper (1995) Breeding Field Crops, 4th Ed., 172-175; Fehr (1987) Principles of Cultivar Development, Vol. 1: Theory and Technique, 360-376).

Insect Resistance Management (IRM) Strategies. Roush *et al.*, for example, outlines two-toxin strategies, also called "pyramiding" or "stacking," for management of insecticidal transgenic crops. (The Royal Society. Phil. Trans. R. Soc. Lond. B. (1998) 353, 1777-1786).

On their website, the United States Environmental Protection Agency (epa.gov/oppbppd1/biopesticides/pips/bt_corn_refuge_2006.htm) publishes the following requirements for providing non-transgenic (*i.e., non-Bt*) refuges (a section of non-Bt crops / corn) for use with transgenic crops producing a single Bt protein active against target pests.

"The specific structured requirements for corn borer-protected Bt (Cry1Ab or Cry1F) corn products are as follows:
Structured refuges: 20% non-Lepidopteran Bt corn refuge in Corn Belt;
   50% non-Lepidopteran Bt refuge in Cotton Belt
Blocks
   Internal (*i.e*., within the Bt field)
   External (*i*.*e*., separate fields within ½ mile (¼ mile if possible)
   of the Bt field to maximize random mating)
In-field Strips
   Strips must be at least 4 rows wide (preferably 6 rows) to
   reduce the effects of larval movement"

In addition, the National Corn Growers Association, on their website:
(ncga.com/insect-resistance-management-fact-sheet-bt-corn)
also provides similar guidance regarding the refuge requirements. For example:
"Requirements of the Corn Borer IRM:
   - Plant at least 20% of your corn acres to refuge hybrids
   - In cotton producing regions, refuge must be 50%
   - Must be planted within 1/2 mile of the refuge hybrids
   - Refuge can be planted as strips within the Bt field; the refuge strips must be at least 4 rows wide
   - Refuge may be treated with conventional pesticides only if economic thresholds are reached for target insect
   - Bt-based sprayable insecticides cannot be used on the refuge corn
   - Appropriate refuge must be planted on every farm with Bt corn"

Similar structured refuge guidelines can be used for FAW-protected Bt crops of the subject invention.

As stated by Roush *et al.* (on pages 1780 and 1784 right column, for example), stacking or pyramiding of two different proteins each effective against the target pests and with little or no cross-resistance can allow for use of a smaller refuge. Roush suggests that for a successful stack, a refuge size of less than 10% refuge, can provide comparable resistance management to about 50% refuge for a single (non-pyramided) trait. For currently available pyramided Bt corn products, the U.S. Environmental Protection Agency requires significantly less (generally 5%) structured refuge of non-Bt corn be planted than for single trait products (generally 20%).

There are various ways of providing the IRM effects of a refuge, including various geometric planting patterns in the fields (as mentioned above) and in-bag seed mixtures, as discussed further by Roush *et al.* (*supra*), and U.S. Patent No. 6,551,962.

The above percentages, or similar refuge ratios, can be used for the subject double or triple stacks or pyramids. For triple stacks with three sites of action against a single target pest, a goal would be zero refuge (or less than 5% refuge, for example). This is particularly true for commercial acreage - of over 10 acres for example.

All patents, patent applications, provisional applications, and publications referred to or cited herein are incorporated by reference in their entirety to the extent they are not inconsistent with the explicit teachings of this specification.

Unless specifically indicated or implied, the terms "a", "an", and "the" signify "at least one" as used herein.

Following are examples that illustrate procedures for practicing the invention. These examples should not be construed as limiting. All percentages are by weight and all solvent mixture proportions are by volume unless otherwise noted. All temperatures are in degrees Celsius.

### EXAMPLES

### Example 1 - ¹²⁵I Labeling of CrylEa and CrylAb Proteins

**Iodination of Cry toxins.** Full-length CrylEa and CrylAb proteins (SEQ ID NOs:1 and 2) were cleaved by trypsin to produce activated, insecticidal forms (as represented by SEQ ID NOS:7 and 8). Purified truncated Cry toxins were was iodinated using Iodo-Beads or Iodo-gen (Pierce). Briefly, two Iodo-Beads were washed twice with 500 µl of phosphate buffered saline, PBS (20 mM sodium phosphate, 0.15 M NaCl, pH 7.5), and placed into a 1.5 ml centrifuge tube behind lead shielding. To this was added 100 µl of PBS. In a hood and through the use of proper radioactive handling techniques, 0.5 mCi Na¹²⁵I (17.4 Ci/mg, Lot 0114, Amersham) was added to the PBS solution with the Iodo-Bead. The components were allowed to react for 5 minutes at room temperature, then 2-25 µg of highly pure truncated Cry protein was added to the solution and allowed to react for an additional 3-5 minutes. The reaction was terminated by removing the solution from the iodo-beads and applying it to a 0.5 ml desalting Zeba spin column (InVitrogen) equilibrated in PBS. The iodo-bead was washed twice with 10 µl of PBS each and the wash solution also applied to the desalting column. The radioactive solution was eluted through the desalting column by centrifugation at 1,000 x g for 2 min.

Radio-purity of the iodinated Cry proteins was determined by SDS-PAGE, phosphorimaging and gamma counting. Briefly, 2 µl of the radioactive protein was separated by SDS-PAGE. After separation, the gels were dried using a BioRad gel drying apparatus following the manufacturer's instructions. The dried gels were imaged by wrapping them in Mylar film (12 µm thick), and exposing them under a Molecular Dynamics storage phosphor screen (35 cm x 43 cm), for 1 hour. The plates were developed using a Molecular Dynamics Storm 820 phosphorimager and the imaged analyzed using ImageQuant ™ software. The radioactive band along with areas immediately above and below the band were cut from the gel using a razor blade and counted in a gamma counter. Radioactivity was only detected in the Cry protein band and in areas below the band. No radioactivity was detected above the band, indicating that all radioactive contaminants consisted of smaller protein components than the truncated Cry protein. These components most probably represent degradation products.

### Example 2 - BBMV Preparation Protocol

**Preparation and Fractionation of Solubilized BBMV's.** Last instar *Spodoptera frugiperda* larvae were fasted overnight and then dissected in the morning after chilling on ice for 15 minutes. The midgut tissue was removed from the body cavity, leaving behind the hindgut attached to the integument. The midgut was placed in 9X volume of ice cold homogenization buffer (300 mM mannitol, 5 mM EGTA, 17 mM tris. base, pH 7.5), supplemented with Protease Inhibitor Cocktail¹ (Sigma P-2714) diluted as recommended by the supplier. The tissue was homogenized with 15 strokes of a glass tissue homogenizer. BBMV's were prepared by the MgCl₂ precipitation method of Wolfersberger (1993). Briefly, an equal volume of a 24 mM MgCl₂ solution in 300 mM mannitol was mixed with the midgut homogenate, stirred for 5 minutes and allowed to stand on ice for 15 min. The solution was centrifuged at 2,500 x g for 15 min at 4° C. The supernatant was saved and the pellet suspended into the original volume of 0.5-X diluted homogenization buffer and centrifuged again. The two supernatants were combined, centrifuged at 27,000 x g for 30 min at 4° C to form the BBMV fraction. The pellet was suspended into 10 ml homogienization buffer and supplemented to protease inhibitiors and centrifuged again at 27,000 x g of r30 min at 4 °C to wash the BBMV's. The resulting pellet was suspended into BBMV Storage Buffer (10 mM HEPES, 130 mM KCl, 10% glycerol, pH 7.4) to a concentration of about 3 mg/ml protein. Protein concentration was determined by using the Bradford method (1976) with bovine serum albumin (BSA) as the standard. Alkaline phosphatase determination was made prior to freezing the samples using the Sigma assay following manufacturer's instructions. The specific activity of this marker enzyme in the BBMV fraction typically increased 7-fold compared to that found in the midgut homogenate fraction. The BBMV's were aliquoted into 250 µl samples, flash frozen in liquid N₂ and stored at -80°C.
¹ Final concentration of cocktail components (in µM) are AEBSF (500), EDTA (250 mM), Bestatin (32), E-64 (0.35), Leupeptin (0.25), and Aprotinin (0.075).

### Example 3 - Method to Measure Binding of ¹²⁵I Cry Proteins to BBMV Proteins

**Binding of ¹²⁵I Cry Proteins to BBMV's.** To determine the optimal amount of BBMV protein to use in the binding assays, a saturation curve was generated. ¹²⁵I radiolabeled Cry protein (0.5 nM) was incubated for 1 hr. at 28 °C with various amounts of BBMV protein, ranging from 0-500 µg/ml in binding buffer (8 mM NaHPO₄, 2 mM KH₂PO₄, 150 mM NaCl, 0.1% bovine serum albumin, pH 7.4). Total volume was 0.5 ml. Bound ¹²⁵I Cry protein was separated from unbound by sampling 150 µl of the reaction mixture in triplicate from a 1.5 ml centrifuge tube into a 500 µl centrifuge tube and centrifuging the samples at 14,000 x g for 6 minutes at room temperature. The supernatant was gently removed, and the pellet gently washed three times with ice cold binding buffer. The bottom of the centrifuge containing the pellet was cut out and placed into a 13 x 75-mm glass culture tube. The samples were counted for 5 minutes each in the gamma counter. The counts contained in the sample were subtracted from background counts (reaction with out any protein) and was plotted versus BBMV protein concentration. The optimal amount of protein to use was determined to be 0.15 mg/ml of BBMV protein.

To determine the binding kinetics, a saturation curve was generated. Briefly, BBMV's (150 µg/ml) were incubated for 1 hr. at 28 °C with increasing concentrations of ¹²⁵I Cry toxin, ranging from 0.01 to 10 nM. Total binding was determined by sampling 150 µl of each concentration in triplicate, centrifugation of the sample and counting as described above. Non-specific binding was determined in the same manner, with the addition of 1,000 nM of the homologous trypsinized non-radioactive Cry toxin (as represented by SEQ ID NOS:7-11, and non-trypsinized SEQ ID NO:6) added to the reaction mixture to saturate all non-specific receptor binding sites. Specific binding was calculated as the difference between total binding and non-specific binding.

Homologous and heterologous competition binding assays were conducted using 150 µg/ml BBMV protein and 0.5 nM of the ¹²⁵I radiolabeled Cry protein. The concentrations of the competitive non-radio labeled Cry toxins added to the reaction mixture ranged from 0.045 to 1,000 nM and were added at the same time as the radioactive ligand, to assure true binding competition. Incubations were carried out for 1 hr. at 28 °C and the amount of ¹²⁵I Cry protein bound to its receptor toxin measured as described above with non-specific binding subtracted. One hundred percent total binding was determined in the absence of any competitor ligand. Results were plotted on a semi-logarithmic plot as percent total specific binding versus concentration of competitive ligand added.

### Example 4 - Summary of Results

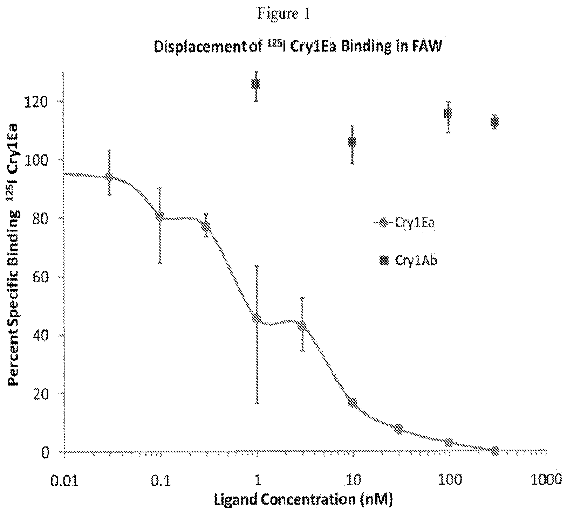

Figure 1 shows percent specific binding of ¹²⁵I Cry1Ea (0.5 nM) in BBMV's from FAW versus competition by unlabeled homologous Cry1Ea (●) and heterologous Cry1Ab (■). The displacement curve for homologous competition by Cry1Ea results in a curve showing 50% displacement of the radioligand at about 1 nM of non-labeled Cry1Ea. CrylAb does not displace the specific binding of ¹²⁵I Cry1Ea at any concentration tested, up to 1,000 nM, or 2,000 times the concentration of ¹²⁵I Cry1Ea used in the assay.

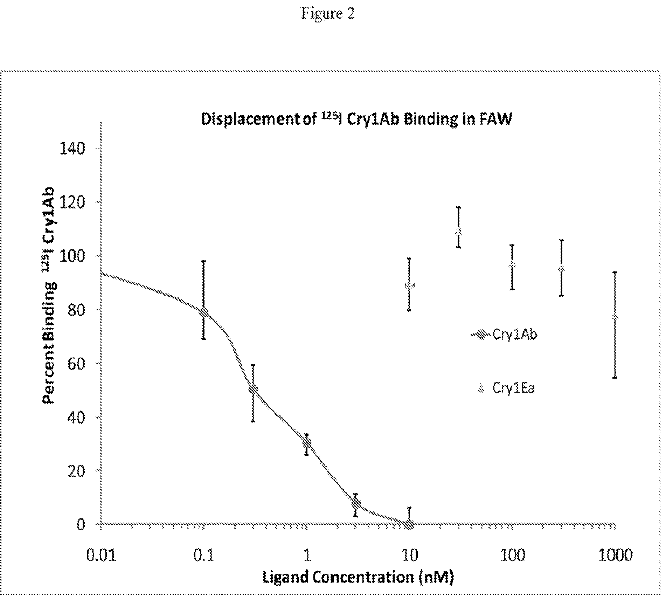

Figure 2 shows the binding of ¹²⁵I Cry1Ab to BBMV's from FAW larvae and its subsequent displacement by increasing concentrations of unlabeled Cry1Ab. Unlabeled Cry1Ab displaces the binding of the radiolabeled Cry1Ab by 50% at a concentration of about 0.3 NM. The binding of I Cry1Ab to FAW BBMV's is not displaced by Cry1Ea. Thus, these two Cry toxins bind at separate sites in the gut of FAW insects.

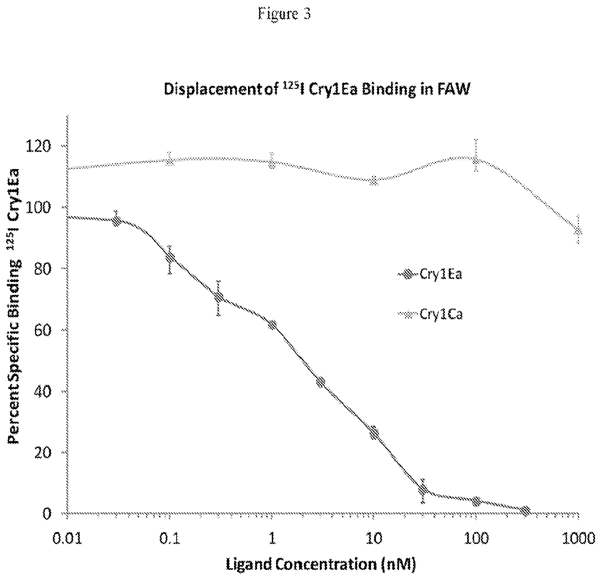

Figure 3 shows percent specific binding of ¹²⁵I Cry1Ea in BBMV's from FAW versus competition by unlabeled homologous Cry1Ea (●) and heterologous Cry1Ca (▲). The displacement curve for homologous competition by Cry1Ea results in a curve showing 50% displacement of the radioligand at about 1 nM of non-labeled Cry1Ea. Cry1Ca was not able to displace the binding of ¹²⁵I Cry1Ea.

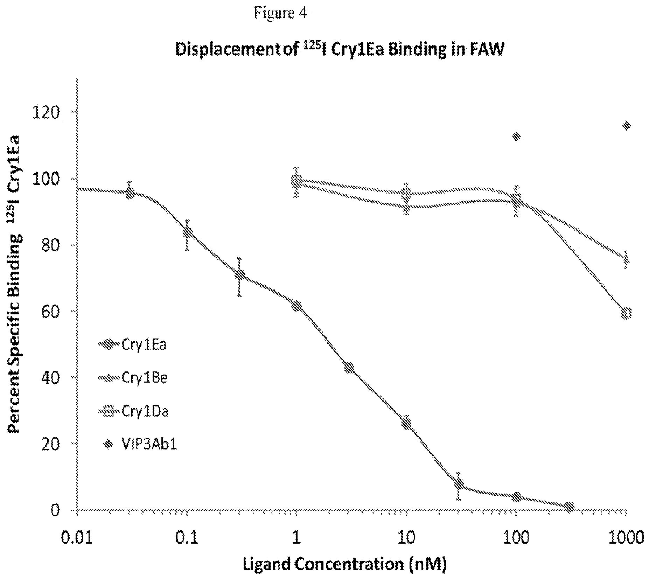

Figure 4 shows the results of a binding assay in which ¹²⁵I Cry1Ea was bound to BBMV's from FAW larvae and Cry1Ea, Cry1Be, Cry1Da, and VIP3Ab1 ligands were subsequently added. Cry1Be, Cry1Da, and VIP3Ab1 were unable to displace the binding of ¹²⁵I Cry1Ea, except for approximately 40% displacement by Cry1Da at 1,000 nM concentration, or 2,000 times the concentration of ¹²⁵I Cry1Ea used in the assay.

### Reference List

Bernardi,R., Tedeschi,G., Ronchi,S., and Palmieri,S. (1996). Isolation and some molecular properties of a trypsin-like enzyme from larvae of European corn borer Ostrinia nubilalis Hnbner (Lepidoptera: pyralidae). Insect Biochemistry and Molecular Biology 26, 883-889.
Christeller,J.T., Laing W.A., Marwick N.P., and Burgess E.P.J. (1992). Midgut protease activities in 12 phytophagous lepidopteran larvae: Dietary and protease inhibitor interactions. Insect Biochem. Molec. Biol. 22, 735-746.
Gatehouse,L.N., Shannon,A.L., Burgess,E.P., and Christeller,J.T. (1997). Characterization of major midgut proteinase cDNAs from Helicoverpa armigera larvae and changes in gene expression in response to four proteinase inhibitors in the diet. Insect Biochem Mol Biol 27, 929-944.
Gomez,I., Arenas,I., Benitez,I., Miranda-Rios,J., Becerril,B., Grande,R., Almagro,J.C., Bravo,A., and Soberon,M. (2006). Specific epitopes of domains II and III of Bacillus thuringiensis Cry1Ab toxin involved in the sequential interaction with cadherin and aminopeptidase-N receptors in Manduca sexta. J Biol. Chem. 281, 34032-34039.
Gomez,I., Dean,D.H., Bravo,A., and Soberon,M. (2003). Molecular basis for Bacillus thuringiensis Cry1Ab toxin specificity: two structural determinants in the Manduca sexta Bt-R1 receptor interact with loops alpha-8 and 2 in domain II of Cy1Ab toxin. Biochemistry 42, 10482-10489.
Gomez,I., Pardo-Lopez,L., Munoz-Garay,C., Fernandez,L.E., Perez,C., Sanchez,J., Soberon,M., and Bravo,A. (2007). Role of receptor interaction in the mode of action of insecticidal Cry and Cyt toxins produced by Bacillus thuringiensis. Peptides 28, 169-173.
Pigott,C.R. and Ellar,D.J. (2007). Role of receptors in Bacillus thuringiensis crystal toxin activity. Microbiol Mol Biol. Rev. 71, 255-281.
Rausell,C., Garcia-Robles,I., Sanchez,J., Munoz-Garay,C., Martinez-Ramirez,A.C., Real,M.D., and Bravo,A. (2004). Role of toxin activation on binding and pore formation activity of the Bacillus thuringiensis Cry3 toxins in membranes of Leptinotarsa decemlineata (Say). Biochim. Biophys Acta 1660, 99-105.
Roush RT. Two-toxin strategies for management of insecticidal transgenic crops: can pyramiding succeed where pesticide mixtures have not? Philos Trans R Soc Lond B Biol Sci. 1998 Oct 29;353(1376):1777-1786.
Van,R.J., Jansens,S., Hofte,H., Degheele,D., and Van,M.H. (1990a). Receptors on the brush border membrane of the insect midgut as determinants of the specificity of Bacillus thuringiensis delta-endotoxins. Appl Environ Microbiol 56, 1378-1385.
Heckel,D.G., Gahan,L.J., Baxter,S.W., Zhao,J.Z., Shelton,A.M., Gould,F., and Tabashnik,B.E. (2007). The diversity of Bt resistance genes in species of Lepidoptera. J Invertebr Pathol 95, 192-197.
Luo,K., Banks,D., and Adang,M.J. (1999). Toxicity, binding, and permeability analyses of four bacillus thuringiensis cry1 delta-endotoxins using brush border membrane vesicles of spodoptera exigua and spodoptera frugiperda. Appl. Environ. Microbiol. 65, 457-464.
Palmer, M., Buchkremer, M, Valeva, A, and Bhakdi, S. Cysteine-specific radioiodination of proteins with fluorescein maleimide. Analytical Biochemistry 253, 175-179. 1997. Ref Type: Journal (Full)
Sambrook,J. and Russell,D.W. (2001). Molecular Cloning: A Laboratory Manual. Cold Spring Harbor Laboratory).
Schlenz, M. L., Babcock, J. M., and Storer, N. P. Response of Cry1F-resistant and Susceptible European Corn Borer and Fall Armyworm Colonies to Cry1A.105 and Cry12Ab2. DAI 0830, 2008. Indianapolis, Dow AgroSciences. Derbi Report.
Sheets, J. J. and Storer, N. P. Analysis of CrylAc Binding to Proteins in Brush Border Membrane Vesicles of Corn Earworm Larvae (Heleothis zea). Interactions with Cry1F Proteins and Its Implication for Resistance in the Field. DAI-0417, 1-26. 2001. Indianapolis, Dow AgroSciences.
Tabashnik,B.E., Liu,Y.B., Finson,N., Masson,L., and Heckel,D.G. (1997). One gene in diamondback moth confers resistance to four Bacillus thuringiensis toxins. Proc. Natl. Acad. Sci. U. S. A 94, 1640-1644.
Tabashnik,B.E., Malvar,T., Liu,Y.B., Finson,N., Borthakur,D., Shin,B.S., Park,S.H., Masson,L., de Maagd,R.A., and Bosch,D. (1996). Cross-resistance of the diamondback moth indicates altered interactions with domain II of Bacillus thuringiensis toxins. Appl. Environ. Microbiol. 62, 2839-2844.
Tabashnik,B.E., Roush,R.T., Earle,E.D., and Shelton,A.M. (2000). Resistance to Bt toxins. Science 287, 42.
Wolfersberger,M.G. (1993). Preparation and partial characterization of amino acid transporting brush border membrane vesicles from the larval midgut of the gypsy moth (Lymantria dispar). Arch. Insect Biochem. Physiol 24, 139-147.
Xu,X., Yu,L., and Wu,Y. (2005). Disruption of a cadherin gene associated with resistance to Cry1Ac {delta}-endotoxin of Bacillus thuringiensis in Helicoverpa armigera. Appl Environ Microbiol 71, 948-954.

The following paragraphs are not claims, but represent preferred aspects and embodiments of the invention.
1. A transgenic plant comprising a polynucleotide encoding a Cry1Ea insecticidal protein, and a second polynucleotide encoding a second insecticidal protein, said second insecticial protein being selected from the group consisting of Cry1Ab, Cry1Be, Cry1Ca, Cry1Da, and Vip3Ab.
2. The transgenic plant of paragraph 1, said plant further comprising DNA encoding a third insecticidal protein.
3. A seed of a plant according to paragraph 1, wherein said seed comprises said heterologous polynucleotides.
4. A seed of a plant according to paragraph 3, wherein said seed comprises said heterologous polynucleotides.
5. A field of plants comprising non-*Bt* refuge plants and a plurality of plants according to paragraph 1, wherein said refuge plants comprise less than 40% of all crop plants in said field.
6. The field of plants of paragraph 5, wherein said refuge plants comprise less than 30% of all the crop plants in said field.
7. The field of plants of paragraph 5, wherein said refuge plants comprise less than 20% of all the crop plants in said field.
8. The field of plants of paragraph 5, wherein said refuge plants comprise less than 10% of all the crop plants in said field.
9. The field of plants of paragraph 5, wherein said refuge plants comprise less than 5% of all the crop plants in said field.
10. The field of plants of paragraph 5, wherein said refuge plants are in blocks or strips.
11. A composition comprising refuge seeds from non-*Bt* refuge plants, and a plurality of seeds of paragraph 3, wherein said refuge seeds comprise less than 40% of all the seeds in the composition.
12. The composition of paragraph 11, wherein said refuge seeds comprise less than 30% of all the seeds in the composition.
13. The composition of paragraph 11, wherein said refuge seeds comprise less than 20% of all the seeds in the composition.
14. The composition of paragraph 11, wherein said refuge seeds comprise less than 10% of all the seeds in the composition.
15. The composition of paragraph 11, wherein said refuge seeds comprise less than 5% of all the seeds in the composition.
16. A method of managing development of resistance to a *Cry* protein by an insect, said method comprising planting seeds to produce a field of plants of paragraph 5.
17. A field of any of paragraphs 5-10, wherein said plants occupy more than 10 acres.
18. A plant of paragraph 1, wherein said plant is selected from the group consisting of corn, soybeans, cotton, canola, and sunflowers.
19. A plant cell of a plant of paragraph 1, wherein said plant cell comprises said polynucleotide encoding said Cry1Ea insecticidal protein, wherein said Cry1Ea insecticidal protein is at least 95% identical with SEQ ID NO:7.
20. A plant of paragraph 1 wherein said Cry1Ea insecticidal protein comprises SEQ ID NO:7.
21. A method of producing the polynucleotide of the plant cell of paragraph 19, said method comprising reproducing said polynucleotide in a plurality of said cells.
22. A method of controlling a fall armyworm insect, said method comprising providing to said insect, for oral ingenstion, a Cry1Ea insecticidal protein and a second insecticidal protein, said second insecticidal protein being selected from the group consisting of Cry1Ab, Cry1Be, Cry1Ca, Cry1Da, and Vip3Ab.
23. A plant cell of a plant of paragraph 1, wherein said second polynucleotide encodes a Cry1Ab insecticidal protein, and wherein said Cry1Ab insecticidal protein is at least 95% identical with SEQ ID NO:8.
24. A plant of paragraph 1 wherein said Cry1Ab insecticidal protein comprises SEQ ID NO:8.
25. A plant cell of a plant of paragraph 1, wherein said plant cell comprises said isolated polynucleotide encoding a Cry1Be insecticidal protein, wherein said Cry1Be insecticidal protein is at least 95% identical with SEQ ID NO:9.
26. A plant of paragraph 1 wherein said Cry1Be insecticidal protein comprises SEQ ID NO:9.
27. A plant cell of a plant of paragraph 1, wherein said plant cell comprises said polynucleotide encoding said Cry1Ca insecticidal protein, wherein said Cry1Ca insecticidal protein is at least 95% identical with SEQ ID NO:10.
28. A plant of paragraph 1 wherein said Cry1Ca insecticidal protein comprises SEQ ID NO:10.
29. A plant cell of a plant of paragraph 1, wherein said plant cell comprises said polynucleotide encoding said Cry1Da insecticidal protein, wherein said Cry1Da insecticidal protein is at least 95% identical with SEQ ID NO:11.
30. A plant of paragraph 1 wherein said Cry1Da insecticidal protein comprises SEQ ID NO:11.
31. A plant cell of a plant of paragraph 1, wherein said plant cell comprises said polynucleotide encoding said Vip3Ab insecticidal protein, wherein said Vip3Ab insecticidal protein is at least 95% identical with SEQ ID NO:6.
32. A plant of paragraph 1 wherein said Vip3Ab insecticidal protein comprises SEQ ID NO:6.
33. The method of paragraph 22, said method comprising providing a third insecticidal protein to said insect.
34. A plant cell of the plant of paragraph 1.
35. A plant cell of the plant of paragraph 2.
36. A plant cell of the plant of paragraph 18.

## Claims

1. A transgenic plant for preventing development of Cry-resistant fall armyworm comprising a polynucleotide encoding a Cry1Ea insecticidal protein comprising SEQ ID NO.7, and a second polynucleotide encoding a Cry1Ca insecticidal protein comprising SEQ ID NO.10, wherein said polynucleotide and said second polynucleotide are heterologous.

2. The transgenic plant of claim 1, said plant further comprising DNA encoding a third insecticidal protein.

3. A seed of the transgenic plant according to claim 1 or 2, wherein said seed comprises said heterologous polynucleotides.

4. The transgenic plant of claim 1, wherein said transgenic plant is selected from the group consisting of corn, soybeans, cotton, canola, and sunflowers.

5. A method of controlling a fall armyworm insect, said method comprising providing to said insect, for oral ingestion, a Cry1Ea insecticidal protein comprising SEQ ID NO.7 and a Cry1Ca insecticidal protein comprising SEQ ID NO.10.

6. The method of claim 5, said method comprising providing a third insecticidal protein to said insect.

7. A transgenic plant for preventing development of Cry-resistant fall armyworm comprising heterologous polynucleotides consisting of a polynucleotide encoding a Cry1Ea insecticidal protein, and a second polynucleotide encoding a Cry1Ca insecticidal protein.

8. A seed of the transgenic plant according to claim 7, wherein said seed comprises said heterologous polynucleotides.

9. The transgenic plant of claim 7, wherein said transgenic plant is selected from the group consisting of corn, soybeans, cotton, canola, and sunflowers.

10. A plant cell of the transgenic plant of claim 7, wherein said plant cell comprises said polynucleotide encoding said Cry1Ea insecticidal protein, wherein said Cry1Ea insecticidal protein is at least 95% identical with SEQ ID NO: 7.

11. The transgenic plant of claim 7 wherein said CrylEa insecticidal protein comprises SEQ ID NO:7.

12. A method of controlling a fall armyworm insect, said method comprising providing to said insect, for oral ingestion, proteins consisting of a Cry1Ea insecticidal protein and a Cry1Ca insecticidal protein.

13. A plant cell of the transgenic plant of claim 7, wherein said plant cell comprises said polynucleotide encoding said Cry1Ca insecticidal protein, wherein said Cry1Ca insecticidal protein is at least 95% identical with SEQ ID NO: 10.

14. The transgenic plant of claim 7 wherein said Cry1Ca insecticidal protein comprises SEQ ID NO: 10.

15. A plant cell of the transgenic plant of claims 1, 2, or 7.


SEQUENCE LISTING

<110>  Dow AgroSciences LLC

<120>  USE OF Cry1Ea IN COMBINATIONS FOR MANAGEMENT OF  RESISTANT FALL
ARMYWORM        INSECTS

<130>  DAS-P0235-US

<160>  11

<170>  PatentIn version 3.5

<210>  1
<211>  1148
<212>  PRT
<213>  Bacillus thuringiensis

<220>
<221>  Cry1Ea
<222>  (1)..(1148)

<400>  1

Met Glu Ile Val Asn Asn Gln Asn Gln Cys Val Pro Tyr Asn Cys Leu
1               5                   10                  15

Asn Asn Pro Glu Asn Glu Ile Leu Asp Ile Glu Arg Ser Asn Ser Thr
            20                  25                  30

Val Ala Thr Asn Ile Ala Leu Glu Ile Ser Arg Leu Leu Ala Ser Ala
        35                  40                  45

Thr Pro Ile Gly Gly Ile Leu Leu Gly Leu Phe Asp Ala Ile Trp Gly
    50                  55                  60

Ser Ile Gly Pro Ser Gln Trp Asp Leu Phe Leu Glu Gln Ile Glu Leu
65                  70                  75                  80

Leu Ile Asp Gln Lys Ile Glu Glu Phe Ala Arg Asn Gln Ala Ile Ser
                85                  90                  95

Arg Leu Glu Gly Ile Ser Ser Leu Tyr Gly Ile Tyr Thr Glu Ala Phe
            100                 105                 110

Arg Glu Trp Glu Ala Asp Pro Thr Asn Pro Ala Leu Lys Glu Glu Met
        115                 120                 125

Arg Thr Gln Phe Asn Asp Met Asn Ser Ile Leu Val Thr Ala Ile Pro
    130                 135                 140

Leu Phe Ser Val Gln Asn Tyr Gln Val Pro Phe Leu Ser Val Tyr Val

145                 150                 155                 160

Gln Ala Ala Asn Leu His Leu Ser Val Leu Arg Asp Val Ser Val Phe
                165                 170                 175

Gly Gln Ala Trp Gly Phe Asp Ile Ala Thr Ile Asn Ser Arg Tyr Asn
            180                 185                 190

Asp Leu Thr Arg Leu Ile Pro Ile Tyr Thr Asp Tyr Ala Val Arg Trp
        195                 200                 205

Tyr Asn Thr Gly Leu Asp Arg Leu Pro Arg Thr Gly Gly Leu Arg Asn
    210                 215                 220

Trp Ala Arg Phe Asn Gln Phe Arg Arg Glu Leu Thr Ile Ser Val Leu
225                 230                 235                 240

Asp Ile Ile Ser Phe Phe Arg Asn Tyr Asp Ser Arg Leu Tyr Pro Ile
                245                 250                 255

Pro Thr Ser Ser Gln Leu Thr Arg Glu Val Tyr Thr Asp Pro Val Ile
            260                 265                 270

Asn Ile Thr Asp Tyr Arg Val Gly Pro Ser Phe Glu Asn Ile Glu Asn
        275                 280                 285

Ser Ala Ile Arg Ser Pro His Leu Met Asp Phe Leu Asn Asn Leu Thr
    290                 295                 300

Ile Asp Thr Asp Leu Ile Arg Gly Val His Tyr Trp Ala Gly His Arg
305                 310                 315                 320

Val Thr Ser His Phe Thr Gly Ser Ser Gln Val Ile Thr Thr Pro Gln
                325                 330                 335

Tyr Gly Ile Thr Ala Asn Ala Glu Pro Arg Arg Thr Ile Ala Pro Ser
            340                 345                 350

Thr Phe Pro Gly Leu Asn Leu Phe Tyr Arg Thr Leu Ser Asn Pro Phe
        355                 360                 365

Phe Arg Arg Ser Glu Asn Ile Thr Pro Thr Leu Gly Ile Asn Val Val
    370                 375                 380

Gln Gly Val Gly Phe Ile Gln Pro Asn Asn Ala Glu Val Leu Tyr Arg
385                 390                 395                 400

Ser Arg Gly Thr Val Asp Ser Leu Asn Glu Leu Pro Ile Asp Gly Glu
                405                 410                 415

Asn Ser Leu Val Gly Tyr Ser His Arg Leu Ser His Val Thr Leu Thr
            420                 425                 430

Arg Ser Leu Tyr Asn Thr Asn Ile Thr Ser Leu Pro Thr Phe Val Trp
        435                 440                 445

Thr His His Ser Ala Thr Asn Thr Asn Thr Ile Asn Pro Asp Ile Ile
    450                 455                 460

Thr Gln Ile Pro Leu Val Lys Gly Phe Arg Leu Gly Gly Gly Thr Ser
465                 470                 475                 480

Val Ile Lys Gly Pro Gly Phe Thr Gly Gly Asp Ile Leu Arg Arg Asn
                485                 490                 495

Thr Ile Gly Glu Phe Val Ser Leu Gln Val Asn Ile Asn Ser Pro Ile
            500                 505                 510

Thr Gln Arg Tyr Arg Leu Arg Phe Arg Tyr Ala Ser Ser Arg Asp Ala
        515                 520                 525

Arg Ile Thr Val Ala Ile Gly Gly Gln Ile Arg Val Asp Met Thr Leu
    530                 535                 540

Glu Lys Thr Met Glu Ile Gly Glu Ser Leu Thr Ser Arg Thr Phe Ser
545                 550                 555                 560

Tyr Thr Asn Phe Ser Asn Pro Phe Ser Phe Arg Ala Asn Pro Asp Ile
                565                 570                 575

Ile Arg Ile Ala Glu Glu Leu Pro Ile Arg Gly Gly Glu Leu Tyr Ile
            580                 585                 590

Asp Lys Ile Glu Leu Ile Leu Ala Asp Ala Thr Leu Glu Ala Glu Ser
        595                 600                 605

Asp Leu Glu Arg Ala Gln Lys Ala Val Asn Ala Leu Phe Thr Ser Ser
    610                 615                 620

Asn Gln Ile Gly Leu Lys Thr Asp Val Thr Asp Tyr His Ile Asp Arg
625                 630                 635                 640

Val Ser Asn Leu Val Glu Cys Leu Ser Asp Glu Phe Cys Leu Asp Glu
                645                 650                 655

Lys Lys Glu Leu Ser Glu Lys Val Lys His Ala Lys Arg Leu Ser Asp
        660                 665                 670

Glu Arg Asn Leu Leu Gln Asp Pro Asn Phe Arg Gly Ile Asn Arg Gln
    675                 680                 685

Leu Asp Arg Gly Trp Arg Gly Ser Thr Asp Ile Thr Ile Gln Gly Gly
    690                 695                 700

Asp Asp Val Phe Lys Glu Asn Tyr Val Thr Leu Leu Gly Thr Phe Asp
705                 710                 715                 720

Glu Cys Tyr Pro Thr Tyr Leu Tyr Gln Lys Ile Asp Glu Ser Lys Leu
                725                 730                 735

Lys Ala Tyr Thr Arg Tyr Gln Leu Arg Gly Tyr Ile Glu Asp Ser Gln
            740                 745                 750

Asp Leu Glu Ile Tyr Leu Ile Arg Tyr Asn Ala Lys His Glu Thr Val
        755                 760                 765

Asn Val Pro Gly Thr Gly Ser Leu Trp Pro Leu Ser Ala Pro Ser Pro
    770                 775                 780

Ile Gly Lys Cys Ala His His Ser His His Phe Ser Leu Asp Ile Asp
785                 790                 795                 800

Val Gly Cys Thr Asp Leu Asn Glu Asp Leu Gly Val Trp Val Ile Phe
                805                 810                 815

Lys Ile Lys Thr Gln Asp Gly His Ala Arg Leu Gly Asn Leu Glu Phe
            820                 825                 830

Leu Glu Glu Lys Pro Leu Val Gly Glu Ala Leu Ala Arg Val Lys Arg
        835                 840                 845

Ala Glu Lys Lys Trp Arg Asp Lys Arg Glu Lys Leu Glu Trp Glu Thr
    850                 855                 860

Asn Ile Val Tyr Lys Glu Ala Lys Glu Ser Val Asp Ala Leu Phe Val
865                 870                 875                 880

Asn Ser Gln Tyr Asp Arg Leu Gln Ala Asp Thr Asn Ile Ala Met Ile
                885                 890                 895

His Ala Ala Asp Lys Arg Val His Ser Ile Arg Glu Ala Tyr Leu Pro
            900                 905                 910

Glu Leu Ser Val Ile Pro Gly Val Asn Ala Ala Ile Phe Glu Glu Leu
        915                 920                 925

Glu Gly Arg Ile Phe Thr Ala Phe Ser Leu Tyr Asp Ala Arg Asn Val
    930                 935                 940

Ile Lys Asn Gly Asp Phe Asn Asn Gly Leu Ser Cys Trp Asn Val Lys
945                 950                 955                 960

Gly His Val Asp Val Glu Glu Gln Asn Asn His Arg Ser Val Leu Val
                965                 970                 975

Val Pro Glu Trp Glu Ala Glu Val Ser Gln Glu Val Arg Val Cys Pro
            980                 985                 990

Gly Arg Gly Tyr Ile Leu Arg Val  Thr Ala Tyr Lys Glu  Gly Tyr Gly
        995                 1000                 1005

Glu Gly  Cys Val Thr Ile His  Glu Ile Glu Asn Asn  Thr Asp Glu
    1010                 1015                 1020

Leu Lys  Phe Ser Asn Cys Val  Glu Glu Glu Val Tyr  Pro Asn Asn
    1025                 1030                 1035

Thr Val  Thr Cys Asn Asp Tyr  Thr Ala Thr Gln Glu  Glu Tyr Glu
    1040                 1045                 1050

Gly Thr  Tyr Thr Ser Arg Asn  Arg Gly Tyr Asp Gly  Ala Tyr Glu
    1055                 1060                 1065

Ser Asn  Ser Ser Val Pro Ala  Asp Tyr Ala Ser Ala  Tyr Glu Glu
    1070                 1075                 1080

Lys Ala  Tyr Thr Asp Gly Arg  Arg Asp Asn Pro Cys  Glu Ser Asn
    1085                 1090                 1095

Arg Gly  Tyr Gly Asp Tyr Thr  Pro Leu Pro Ala Gly  Tyr Val Thr
    1100                 1105                 1110

Lys Glu  Leu Glu Tyr Phe Pro  Glu Thr Asp Lys Val  Trp Ile Glu
    1115                 1120                 1125

Ile Gly  Glu Thr Glu Gly Thr  Phe Ile Val Asp Ser  Val Glu Leu
    1130                 1135                 1140

Leu Leu  Met Glu Glu

    1145

<210>  2
<211>  1155
<212>  PRT
<213>  Bacillus thuringiensis

<220>
<221>  Cry1Ab
<222>  (1)..(1155)

<400>  2

Met Asp Asn Asn Pro Asn Ile Asn Glu Cys Ile Pro Tyr Asn Cys Leu
1               5                   10                  15

Ser Asn Pro Glu Val Glu Val Leu Gly Gly Glu Arg Ile Glu Thr Gly
            20                  25                  30

Tyr Thr Pro Ile Asp Ile Ser Leu Ser Leu Thr Gln Phe Leu Leu Ser
        35                  40                  45

Glu Phe Val Pro Gly Ala Gly Phe Val Leu Gly Leu Val Asp Ile Ile
    50                  55                  60

Trp Gly Ile Phe Gly Pro Ser Gln Trp Asp Ala Phe Leu Val Gln Ile
65                  70                  75                  80

Glu Gln Leu Ile Asn Gln Arg Ile Glu Glu Phe Ala Arg Asn Gln Ala
                85                  90                  95

Ile Ser Arg Leu Glu Gly Leu Ser Asn Leu Tyr Gln Ile Tyr Ala Glu
            100                 105                 110

Ser Phe Arg Glu Trp Glu Ala Asp Pro Thr Asn Pro Ala Leu Arg Glu
        115                 120                 125

Glu Met Arg Ile Gln Phe Asn Asp Met Asn Ser Ala Leu Thr Thr Ala
    130                 135                 140

Ile Pro Leu Phe Ala Val Gln Asn Tyr Gln Val Pro Leu Leu Ser Val
145                 150                 155                 160

Tyr Val Gln Ala Ala Asn Leu His Leu Ser Val Leu Arg Asp Val Ser
                165                 170                 175

Val Phe Gly Gln Arg Trp Gly Phe Asp Ala Ala Thr Ile Asn Ser Arg
            180                 185                 190

Tyr Asn Asp Leu Thr Arg Leu Ile Gly Asn Tyr Thr Asp His Ala Val
        195                 200                 205

Arg Trp Tyr Asn Thr Gly Leu Glu Arg Val Trp Gly Pro Asp Ser Arg
    210                 215                 220

Asp Trp Ile Arg Tyr Asn Gln Phe Arg Arg Glu Leu Thr Leu Thr Val
225                 230                 235                 240

Leu Asp Ile Val Ser Leu Phe Pro Asn Tyr Asp Ser Arg Thr Tyr Pro
                245                 250                 255

Ile Arg Thr Val Ser Gln Leu Thr Arg Glu Ile Tyr Thr Asn Pro Val
            260                 265                 270

Leu Glu Asn Phe Asp Gly Ser Phe Arg Gly Ser Ala Gln Gly Ile Glu
        275                 280                 285

Gly Ser Ile Arg Ser Pro His Leu Met Asp Ile Leu Asn Ser Ile Thr
    290                 295                 300

Ile Tyr Thr Asp Ala His Arg Gly Glu Tyr Tyr Trp Ser Gly His Gln
305                 310                 315                 320

Ile Met Ala Ser Pro Val Gly Phe Ser Gly Pro Glu Phe Thr Phe Pro
                325                 330                 335

Leu Tyr Gly Thr Met Gly Asn Ala Ala Pro Gln Gln Arg Ile Val Ala
            340                 345                 350

Gln Leu Gly Gln Gly Val Tyr Arg Thr Leu Ser Ser Thr Leu Tyr Arg
        355                 360                 365

Arg Pro Phe Asn Ile Gly Ile Asn Asn Gln Gln Leu Ser Val Leu Asp
    370                 375                 380

Gly Thr Glu Phe Ala Tyr Gly Thr Ser Ser Asn Leu Pro Ser Ala Val
385                 390                 395                 400

Tyr Arg Lys Ser Gly Thr Val Asp Ser Leu Asp Glu Ile Pro Pro Gln
                405                 410                 415

Asn Asn Asn Val Pro Pro Arg Gln Gly Phe Ser His Arg Leu Ser His
            420                 425                 430

Val Ser Met Phe Arg Ser Gly Phe Ser Asn Ser Ser Val Ser Ile Ile
        435                 440                 445

Arg Ala Pro Met Phe Ser Trp Ile His Arg Ser Ala Glu Phe Asn Asn
    450                 455                 460

Ile Ile Pro Ser Ser Gln Ile Thr Gln Ile Pro Leu Thr Lys Ser Thr
465                 470                 475                 480

Asn Leu Gly Ser Gly Thr Ser Val Val Lys Gly Pro Gly Phe Thr Gly
                485                 490                 495

Gly Asp Ile Leu Arg Arg Thr Ser Pro Gly Gln Ile Ser Thr Leu Arg
            500                 505                 510

Val Asn Ile Thr Ala Pro Leu Ser Gln Arg Tyr Arg Val Arg Ile Arg
        515                 520                 525

Tyr Ala Ser Thr Thr Asn Leu Gln Phe His Thr Ser Ile Asp Gly Arg
    530                 535                 540

Pro Ile Asn Gln Gly Asn Phe Ser Ala Thr Met Ser Ser Gly Ser Asn
545                 550                 555                 560

Leu Gln Ser Gly Ser Phe Arg Thr Val Gly Phe Thr Thr Pro Phe Asn
                565                 570                 575

Phe Ser Asn Gly Ser Ser Val Phe Thr Leu Ser Ala His Val Phe Asn
            580                 585                 590

Ser Gly Asn Glu Val Tyr Ile Asp Arg Ile Glu Phe Val Pro Ala Glu
        595                 600                 605

Val Thr Leu Glu Ala Glu Ser Asp Leu Glu Arg Ala Gln Lys Ala Val
    610                 615                 620

Asn Ala Leu Phe Thr Ser Ser Asn Gln Ile Gly Leu Lys Thr Asp Val
625                 630                 635                 640

Thr Asp Tyr His Ile Asp Arg Val Ser Asn Leu Val Glu Cys Leu Ser
                645                 650                 655

Asp Glu Phe Cys Leu Asp Glu Lys Lys Glu Leu Ser Glu Lys Val Lys
            660                 665                 670

His Ala Lys Arg Leu Ser Asp Glu Arg Asn Leu Leu Gln Asp Pro Asn
        675                 680                 685

Phe Arg Gly Ile Asn Arg Gln Leu Asp Arg Gly Trp Arg Gly Ser Thr
    690                 695                 700

Asp Ile Thr Ile Gln Gly Gly Asp Asp Val Phe Lys Glu Asn Tyr Val
705                 710                 715                 720

Thr Leu Leu Gly Thr Phe Asp Glu Cys Tyr Pro Thr Tyr Leu Tyr Gln
                725                 730                 735

Lys Ile Asp Glu Ser Lys Leu Lys Ala Tyr Thr Arg Tyr Gln Leu Arg
            740                 745                 750

Gly Tyr Ile Glu Asp Ser Gln Asp Leu Glu Ile Tyr Leu Ile Arg Tyr
        755                 760                 765

Asn Ala Lys His Glu Thr Val Asn Val Pro Gly Thr Gly Ser Leu Trp
    770                 775                 780

Pro Leu Ser Ala Pro Ser Pro Ile Gly Lys Cys Ala His His Ser His
785                 790                 795                 800

His Phe Ser Leu Asp Ile Asp Val Gly Cys Thr Asp Leu Asn Glu Asp
                805                 810                 815

Leu Gly Val Trp Val Ile Phe Lys Ile Lys Thr Gln Asp Gly His Ala
            820                 825                 830

Arg Leu Gly Asn Leu Glu Phe Leu Glu Glu Lys Pro Leu Val Gly Glu
        835                 840                 845

Ala Leu Ala Arg Val Lys Arg Ala Glu Lys Lys Trp Arg Asp Lys Arg
    850                 855                 860

Glu Lys Leu Glu Trp Glu Thr Asn Ile Val Tyr Lys Glu Ala Lys Glu
865                 870                 875                 880

Ser Val Asp Ala Leu Phe Val Asn Ser Gln Tyr Asp Arg Leu Gln Ala
                885                 890                 895

Asp Thr Asn Ile Ala Met Ile His Ala Ala Asp Lys Arg Val His Ser
            900                 905                 910

Ile Arg Glu Ala Tyr Leu Pro Glu Leu Ser Val Ile Pro Gly Val Asn
        915                 920                 925

Ala Ala Ile Phe Glu Glu Leu Glu Gly Arg Ile Phe Thr Ala Phe Ser
    930                 935                 940

Leu Tyr Asp Ala Arg Asn Val Ile Lys Asn Gly Asp Phe Asn Asn Gly

945                 950                 955                 960

Leu Ser Cys Trp Asn Val Lys Gly His Val Asp Val Glu Glu Gln Asn
                965                 970                 975

Asn His Arg Ser Val Leu Val Val Pro Glu Trp Glu Ala Glu Val Ser
            980                 985                 990

Gln Glu Val Arg Val Cys Pro Gly  Arg Gly Tyr Ile Leu  Arg Val Thr
        995                 1000                 1005

Ala Tyr  Lys Glu Gly Tyr Gly  Glu Gly Cys Val Thr  Ile His Glu
    1010                 1015                 1020

Ile Glu  Asn Asn Thr Asp Glu  Leu Lys Phe Ser Asn  Cys Val Glu
    1025                 1030                 1035

Glu Glu  Val Tyr Pro Asn Asn  Thr Val Thr Cys Asn  Asp Tyr Thr
    1040                 1045                 1050

Ala Thr  Gln Glu Glu Tyr Glu  Gly Thr Tyr Thr Ser  Arg Asn Arg
    1055                 1060                 1065

Gly Tyr  Asp Gly Ala Tyr Glu  Ser Asn Ser Ser Val  Pro Ala Asp
    1070                 1075                 1080

Tyr Ala  Ser Ala Tyr Glu Glu  Lys Ala Tyr Thr Asp  Gly Arg Arg
    1085                 1090                 1095

Asp Asn  Pro Cys Glu Ser Asn  Arg Gly Tyr Gly Asp  Tyr Thr Pro
    1100                 1105                 1110

Leu Pro  Ala Gly Tyr Val Thr  Lys Glu Leu Glu Tyr  Phe Pro Glu
    1115                 1120                 1125

Thr Asp  Lys Val Trp Ile Glu  Ile Gly Glu Thr Glu  Gly Thr Phe
    1130                 1135                 1140

Ile Val  Asp Ser Val Glu Leu  Leu Leu Met Glu Glu
    1145                 1150                 1155

<210>  3
<211>  1186
<212>  PRT
<213>  Bacillus thuringiensis

<220>
<221>  Cry1Be

<222>  (1)..(1186)

<400>  3

Met Thr Ser Asn Arg Lys Asn Glu Asn Glu Ile Ile Asn Ala Leu Ser
1               5                   10                  15

Ile Pro Ala Val Ser Asn His Ser Ala Gln Met Asn Leu Ser Thr Asp
            20                  25                  30

Ala Arg Ile Glu Asp Ser Leu Cys Ile Ala Glu Gly Asn Asn Ile Asp
        35                  40                  45

Pro Phe Val Ser Ala Ser Thr Val Gln Thr Gly Ile Asn Ile Ala Gly
    50                  55                  60

Arg Ile Leu Gly Val Leu Gly Val Pro Phe Ala Gly Gln Ile Ala Ser
65                  70                  75                  80

Phe Tyr Ser Phe Leu Val Gly Glu Leu Trp Pro Arg Gly Arg Asp Pro
                85                  90                  95

Trp Glu Ile Phe Leu Glu His Val Glu Gln Leu Ile Arg Gln Gln Val
            100                 105                 110

Thr Glu Asn Thr Arg Asp Thr Ala Leu Ala Arg Leu Gln Gly Leu Gly
        115                 120                 125

Asn Ser Phe Arg Ala Tyr Gln Gln Ser Leu Glu Asp Trp Leu Glu Asn
    130                 135                 140

Arg Asp Asp Ala Arg Thr Arg Ser Val Leu Tyr Thr Gln Tyr Ile Ala
145                 150                 155                 160

Leu Glu Leu Asp Phe Leu Asn Ala Met Pro Leu Phe Ala Ile Arg Asn
                165                 170                 175

Gln Glu Val Pro Leu Leu Met Val Tyr Ala Gln Ala Ala Asn Leu His
            180                 185                 190

Leu Leu Leu Leu Arg Asp Ala Ser Leu Phe Gly Ser Glu Phe Gly Leu
        195                 200                 205

Thr Ser Gln Glu Ile Gln Arg Tyr Tyr Glu Arg Gln Val Glu Lys Thr
    210                 215                 220

Arg Glu Tyr Ser Asp Tyr Cys Ala Arg Trp Tyr Asn Thr Gly Leu Asn
225                 230                 235                 240

Asn Leu Arg Gly Thr Asn Ala Glu Ser Trp Leu Arg Tyr Asn Gln Phe
                245                 250                 255

Arg Arg Asp Leu Thr Leu Gly Val Leu Asp Leu Val Ala Leu Phe Pro
            260                 265                 270

Ser Tyr Asp Thr Arg Val Tyr Pro Met Asn Thr Ser Ala Gln Leu Thr
        275                 280                 285

Arg Glu Ile Tyr Thr Asp Pro Ile Gly Arg Thr Asn Ala Pro Ser Gly
    290                 295                 300

Phe Ala Ser Thr Asn Trp Phe Asn Asn Asn Ala Pro Ser Phe Ser Ala
305                 310                 315                 320

Ile Glu Ala Ala Val Ile Arg Pro Pro His Leu Leu Asp Phe Pro Glu
                325                 330                 335

Gln Leu Thr Ile Phe Ser Val Leu Ser Arg Trp Ser Asn Thr Gln Tyr
            340                 345                 350

Met Asn Tyr Trp Val Gly His Arg Leu Glu Ser Arg Thr Ile Arg Gly
        355                 360                 365

Ser Leu Ser Thr Ser Thr His Gly Asn Thr Asn Thr Ser Ile Asn Pro
    370                 375                 380

Val Thr Leu Gln Phe Thr Ser Arg Asp Val Tyr Arg Thr Glu Ser Phe
385                 390                 395                 400

Ala Gly Ile Asn Ile Leu Leu Thr Thr Pro Val Asn Gly Val Pro Trp
                405                 410                 415

Ala Arg Phe Asn Trp Arg Asn Pro Leu Asn Ser Leu Arg Gly Ser Leu
            420                 425                 430

Leu Tyr Thr Ile Gly Tyr Thr Gly Val Gly Thr Gln Leu Phe Asp Ser
        435                 440                 445

Glu Thr Glu Leu Pro Pro Glu Thr Thr Glu Arg Pro Asn Tyr Glu Ser
    450                 455                 460

Tyr Ser His Arg Leu Ser Asn Ile Arg Leu Ile Ser Gly Asn Thr Leu
465                 470                 475                 480

Arg Ala Pro Val Tyr Ser Trp Thr His Arg Ser Ala Asp Arg Thr Asn
                485                 490                 495

Thr Ile Ser Ser Asp Ser Ile Thr Gln Ile Pro Leu Val Lys Ser Phe
            500                 505                 510

Asn Leu Asn Ser Gly Thr Ser Val Val Ser Gly Pro Gly Phe Thr Gly
        515                 520                 525

Gly Asp Ile Ile Arg Thr Asn Val Asn Gly Ser Val Leu Ser Met Gly
    530                 535                 540

Leu Asn Phe Asn Asn Thr Ser Leu Gln Arg Tyr Arg Val Arg Val Arg
545                 550                 555                 560

Tyr Ala Ala Ser Gln Thr Met Val Leu Arg Val Thr Val Gly Gly Ser
                565                 570                 575

Thr Thr Phe Asp Gln Gly Phe Pro Ser Thr Met Ser Ala Asn Glu Ser
            580                 585                 590

Leu Thr Ser Gln Ser Phe Arg Phe Ala Glu Phe Pro Val Gly Ile Ser
        595                 600                 605

Ala Ser Gly Ser Gln Thr Ala Gly Ile Ser Ile Ser Asn Asn Ala Gly
    610                 615                 620

Arg Gln Thr Phe His Phe Asp Lys Ile Glu Phe Ile Pro Ile Thr Ala
625                 630                 635                 640

Thr Leu Glu Ala Glu Ser Asp Leu Glu Arg Ala Gln Lys Ala Val Asn
                645                 650                 655

Ala Leu Phe Thr Ser Ser Asn Gln Ile Gly Leu Lys Thr Asp Val Thr
            660                 665                 670

Asp Tyr His Ile Asp Arg Val Ser Asn Leu Val Glu Cys Leu Ser Asp
        675                 680                 685

Glu Phe Cys Leu Asp Glu Lys Lys Glu Leu Ser Glu Lys Val Lys His
    690                 695                 700

Ala Lys Arg Leu Ser Asp Glu Arg Asn Leu Leu Gln Asp Pro Asn Phe
705                 710                 715                 720

Arg Gly Ile Asn Arg Gln Leu Asp Arg Gly Trp Arg Gly Ser Thr Asp
                725                 730                 735

Ile Thr Ile Gln Gly Gly Asp Asp Val Phe Lys Glu Asn Tyr Val Thr

740                 745                 750

Leu Leu Gly Thr Phe Asp Glu Cys Tyr Pro Thr Tyr Leu Tyr Gln Lys
        755                 760                 765

Ile Asp Glu Ser Lys Leu Lys Ala Tyr Thr Arg Tyr Gln Leu Arg Gly
    770                 775                 780

Tyr Ile Glu Asp Ser Gln Asp Leu Glu Ile Tyr Leu Ile Arg Tyr Asn
785                 790                 795                 800

Ala Lys His Glu Thr Val Asn Val Pro Gly Thr Gly Ser Leu Trp Pro
                805                 810                 815

Leu Ser Ala Pro Ser Pro Ile Gly Lys Cys Ala His His Ser His His
            820                 825                 830

Phe Ser Leu Asp Ile Asp Val Gly Cys Thr Asp Leu Asn Glu Asp Leu
        835                 840                 845

Gly Val Trp Val Ile Phe Lys Ile Lys Thr Gln Asp Gly His Ala Arg
    850                 855                 860

Leu Gly Asn Leu Glu Phe Leu Glu Glu Lys Pro Leu Val Gly Glu Ala
865                 870                 875                 880

Leu Ala Arg Val Lys Arg Ala Glu Lys Lys Trp Arg Asp Lys Arg Glu
                885                 890                 895

Lys Leu Glu Trp Glu Thr Asn Ile Val Tyr Lys Glu Ala Lys Glu Ser
            900                 905                 910

Val Asp Ala Leu Phe Val Asn Ser Gln Tyr Asp Arg Leu Gln Ala Asp
        915                 920                 925

Thr Asn Ile Ala Met Ile His Ala Ala Asp Lys Arg Val His Ser Ile
    930                 935                 940

Arg Glu Ala Tyr Leu Pro Glu Leu Ser Val Ile Pro Gly Val Asn Ala
945                 950                 955                 960

Ala Ile Phe Glu Glu Leu Glu Gly Arg Ile Phe Thr Ala Phe Ser Leu
                965                 970                 975

Tyr Asp Ala Arg Asn Val Ile Lys Asn Gly Asp Phe Asn Asn Gly Leu
            980                 985                 990

Ser Cys Trp Asn Val Lys Gly His  Val Asp Val Glu Glu  Gln Asn Asn
        995                 1000                1005

His Arg  Ser Val Leu Val Val  Pro Glu Trp Glu Ala  Glu Val Ser
    1010                 1015                1020

Gln Glu  Val Arg Val Cys Pro  Gly Arg Gly Tyr Ile  Leu Arg Val
    1025                 1030                1035

Thr Ala  Tyr Lys Glu Gly Tyr  Gly Glu Gly Cys Val  Thr Ile His
    1040                 1045                1050

Glu Ile  Glu Asn Asn Thr Asp  Glu Leu Lys Phe Ser  Asn Cys Val
    1055                 1060                1065

Glu Glu  Glu Val Tyr Pro Asn  Asn Thr Val Thr Cys  Asn Asp Tyr
    1070                 1075                1080

Thr Ala  Thr Gln Glu Glu Tyr  Glu Gly Thr Tyr Thr  Ser Arg Asn
    1085                 1090                1095

Arg Gly  Tyr Asp Gly Ala Tyr  Glu Ser Asn Ser Ser  Val Pro Ala
    1100                 1105                1110

Asp Tyr  Ala Ser Ala Tyr Glu  Glu Lys Ala Tyr Thr  Asp Gly Arg
    1115                 1120                1125

Arg Asp  Asn Pro Cys Glu Ser  Asn Arg Gly Tyr Gly  Asp Tyr Thr
    1130                 1135                1140

Pro Leu  Pro Ala Gly Tyr Val  Thr Lys Glu Leu Glu  Tyr Phe Pro
    1145                 1150                1155

Glu Thr  Asp Lys Val Trp Ile  Glu Ile Gly Glu Thr  Glu Gly Thr
    1160                 1165                1170

Phe Ile  Val Asp Ser Val Glu  Leu Leu Leu Met Glu  Glu
    1175                 1180                1185

<210>  4
<211>  1164
<212>  PRT
<213>  Bacillus thuringiensis

<220>
<221>  Cry1Ca
<222>  (1)..(1164)

<400>  4

Met Asp Asn Asn Pro Asn Ile Asn Glu Cys Ile Pro Tyr Asn Cys Leu
1               5                   10                  15

Ser Asn Pro Glu Glu Val Leu Leu Asp Gly Glu Arg Ile Ser Thr Gly
            20                  25                  30

Asn Ser Ser Ile Asp Ile Ser Leu Ser Leu Val Gln Phe Leu Val Ser
        35                  40                  45

Asn Phe Val Pro Gly Gly Gly Phe Leu Val Gly Leu Ile Asp Phe Val
    50                  55                  60

Trp Gly Ile Val Gly Pro Ser Gln Trp Asp Ala Phe Leu Val Gln Ile
65                  70                  75                  80

Glu Gln Leu Ile Asn Glu Arg Ile Ala Glu Phe Ala Arg Asn Ala Ala
                85                  90                  95

Ile Ala Asn Leu Glu Gly Leu Gly Asn Asn Phe Asn Ile Tyr Val Glu
            100                 105                 110

Ala Phe Lys Glu Trp Glu Glu Asp Pro Lys Asn Pro Ala Thr Arg Thr
        115                 120                 125

Arg Val Ile Asp Arg Phe Arg Ile Leu Asp Gly Leu Leu Glu Arg Asp
    130                 135                 140

Ile Pro Ser Phe Arg Ile Ser Gly Phe Glu Val Pro Leu Leu Ser Val
145                 150                 155                 160

Tyr Ala Gln Ala Ala Asn Leu His Leu Ala Ile Leu Arg Asp Ser Val
                165                 170                 175

Ile Phe Gly Glu Arg Trp Gly Leu Thr Thr Ile Asn Val Asn Glu Asn
            180                 185                 190

Tyr Asn Arg Leu Ile Arg His Ile Asp Glu Tyr Ala Asp His Cys Ala
        195                 200                 205

Asn Thr Tyr Asn Arg Gly Leu Asn Asn Leu Pro Lys Ser Thr Tyr Gln
    210                 215                 220

Asp Trp Ile Thr Tyr Asn Arg Leu Arg Arg Asp Leu Thr Leu Thr Val
225                 230                 235                 240

Leu Asp Ile Ala Ala Phe Phe Pro Asn Tyr Asp Asn Arg Arg Tyr Pro
                245                 250                 255

Ile Gln Pro Val Gly Gln Leu Thr Arg Glu Val Tyr Thr Asp Pro Leu
            260                 265                 270

Ile Asn Phe Asn Pro Gln Leu Gln Ser Val Ala Gln Leu Pro Thr Phe
        275                 280                 285

Asn Val Met Glu Asn Ser Ala Ile Arg Asn Pro His Leu Phe Asp Ile
    290                 295                 300

Leu Asn Asn Leu Thr Ile Phe Thr Asp Trp Phe Ser Val Gly Arg Asn
305                 310                 315                 320

Phe Tyr Trp Gly Gly His Arg Val Ile Ser Ser Leu Ile Gly Gly Gly
                325                 330                 335

Asn Ile Thr Ser Pro Ile Tyr Gly Arg Glu Ala Asn Gln Glu Pro Pro
            340                 345                 350

Arg Ser Phe Thr Phe Asn Gly Pro Val Phe Arg Thr Leu Ser Asn Pro
        355                 360                 365

Thr Leu Arg Leu Leu Gln Gln Pro Trp Pro Ala Pro Pro Phe Asn Leu
    370                 375                 380

Arg Gly Val Glu Gly Val Glu Phe Ser Thr Pro Thr Asn Ser Phe Thr
385                 390                 395                 400

Tyr Arg Gly Arg Gly Thr Val Asp Ser Leu Thr Glu Leu Pro Pro Glu
                405                 410                 415

Asp Asn Ser Val Pro Pro Arg Glu Gly Tyr Ser His Arg Leu Cys His
            420                 425                 430

Ala Thr Phe Val Gln Arg Ser Gly Thr Pro Phe Leu Thr Thr Gly Val
        435                 440                 445

Val Phe Ser Trp Thr His Arg Ser Ala Thr Leu Thr Asn Thr Ile Asp
    450                 455                 460

Pro Glu Arg Ile Asn Gln Ile Pro Leu Val Lys Gly Phe Arg Val Trp
465                 470                 475                 480

Gly Gly Thr Ser Val Ile Thr Gly Pro Gly Phe Thr Gly Gly Asp Ile
                485                 490                 495

Leu Arg Arg Asn Thr Phe Gly Asp Phe Val Ser Leu Gln Val Asn Ile

            500                 505                 510

Asn Ser Pro Ile Thr Gln Arg Tyr Arg Leu Arg Phe Arg Tyr Ala Ser
        515                 520                 525

Ser Arg Asp Ala Arg Val Ile Val Leu Thr Gly Ala Ala Ser Thr Gly
    530                 535                 540

Val Gly Gly Gln Val Ser Val Asn Met Pro Leu Gln Lys Thr Met Glu
545                 550                 555                 560

Ile Gly Glu Asn Leu Thr Ser Arg Thr Phe Arg Tyr Thr Asp Phe Ser
                565                 570                 575

Asn Pro Phe Ser Phe Arg Ala Asn Pro Asp Ile Ile Gly Ile Ser Glu
            580                 585                 590

Gln Pro Leu Phe Gly Ala Gly Ser Ile Ser Ser Gly Glu Leu Tyr Ile
        595                 600                 605

Asp Lys Ile Glu Ile Ile Leu Ala Asp Ala Thr Leu Glu Ala Glu Ser
    610                 615                 620

Asp Leu Glu Arg Ala Gln Lys Ala Val Asn Ala Leu Phe Thr Ser Ser
625                 630                 635                 640

Asn Gln Ile Gly Leu Lys Thr Asp Val Thr Asp Tyr His Ile Asp Arg
                645                 650                 655

Val Ser Asn Leu Val Glu Cys Leu Ser Asp Glu Phe Cys Leu Asp Glu
            660                 665                 670

Lys Lys Glu Leu Ser Glu Lys Val Lys His Ala Lys Arg Leu Ser Asp
        675                 680                 685

Glu Arg Asn Leu Leu Gln Asp Pro Asn Phe Arg Gly Ile Asn Arg Gln
    690                 695                 700

Leu Asp Arg Gly Trp Arg Gly Ser Thr Asp Ile Thr Ile Gln Gly Gly
705                 710                 715                 720

Asp Asp Val Phe Lys Glu Asn Tyr Val Thr Leu Leu Gly Thr Phe Asp
                725                 730                 735

Glu Cys Tyr Pro Thr Tyr Leu Tyr Gln Lys Ile Asp Glu Ser Lys Leu
            740                 745                 750

Lys Ala Tyr Thr Arg Tyr Gln Leu Arg Gly Tyr Ile Glu Asp Ser Gln
        755                 760                 765

Asp Leu Glu Ile Tyr Leu Ile Arg Tyr Asn Ala Lys His Glu Thr Val
    770                 775                 780

Asn Val Pro Gly Thr Gly Ser Leu Trp Pro Leu Ser Ala Pro Ser Pro
785                 790                 795                 800

Ile Gly Lys Cys Ala His His Ser His His Phe Ser Leu Asp Ile Asp
                805                 810                 815

Val Gly Cys Thr Asp Leu Asn Glu Asp Leu Gly Val Trp Val Ile Phe
            820                 825                 830

Lys Ile Lys Thr Gln Asp Gly His Ala Arg Leu Gly Asn Leu Glu Phe
        835                 840                 845

Leu Glu Glu Lys Pro Leu Val Gly Glu Ala Leu Ala Arg Val Lys Arg
    850                 855                 860

Ala Glu Lys Lys Trp Arg Asp Lys Arg Glu Lys Leu Glu Trp Glu Thr
865                 870                 875                 880

Asn Ile Val Tyr Lys Glu Ala Lys Glu Ser Val Asp Ala Leu Phe Val
                885                 890                 895

Asn Ser Gln Tyr Asp Arg Leu Gln Ala Asp Thr Asn Ile Ala Met Ile
            900                 905                 910

His Ala Ala Asp Lys Arg Val His Ser Ile Arg Glu Ala Tyr Leu Pro
        915                 920                 925

Glu Leu Ser Val Ile Pro Gly Val Asn Ala Ala Ile Phe Glu Glu Leu
    930                 935                 940

Glu Gly Arg Ile Phe Thr Ala Phe Ser Leu Tyr Asp Ala Arg Asn Val
945                 950                 955                 960

Ile Lys Asn Gly Asp Phe Asn Asn Gly Leu Ser Cys Trp Asn Val Lys
                965                 970                 975

Gly His Val Asp Val Glu Glu Gln Asn Asn His Arg Ser Val Leu Val
            980                 985                 990

Val Pro Glu Trp Glu Ala Glu Val  Ser Gln Glu Val Arg  Val Cys Pro
        995                 1000                1005

Gly Arg Gly Tyr Ile Leu Arg Val Thr Ala Tyr Lys Glu Gly Tyr
    1010                1015                1020

Gly Glu Gly Cys Val Thr Ile His Glu Ile Glu Asn Asn Thr Asp
    1025                1030                1035

Glu Leu Lys Phe Ser Asn Cys Val Glu Glu Glu Val Tyr Pro Asn
    1040                1045                1050

Asn Thr Val Thr Cys Asn Asp Tyr Thr Ala Thr Gln Glu Glu Tyr
    1055                1060                1065

Glu Gly Thr Tyr Thr Ser Arg Asn Arg Gly Tyr Asp Gly Ala Tyr
    1070                1075                1080

Glu Ser Asn Ser Ser Val Pro Ala Asp Tyr Ala Ser Ala Tyr Glu
    1085                1090                1095

Glu Lys Ala Tyr Thr Asp Gly Arg Arg Asp Asn Pro Cys Glu Ser
    1100                1105                1110

Asn Arg Gly Tyr Gly Asp Tyr Thr Pro Leu Pro Ala Gly Tyr Val
    1115                1120                1125

Thr Lys Glu Leu Glu Tyr Phe Pro Glu Thr Asp Lys Val Trp Ile
    1130                1135                1140

Glu Ile Gly Glu Thr Glu Gly Thr Phe Ile Val Asp Ser Val Glu
    1145                1150                1155

Leu Leu Leu Met Glu Glu
    1160

<210>  5
<211>  1139
<212>  PRT
<213>  Bacillus thuringiensis

<220>
<221>  Cry1Da
<222>  (1)..(1139)

<400>  5

Met Glu Ile Asn Asn Gln Asn Gln Cys Val Pro Tyr Asn Cys Leu Ser
1               5                   10                  15

Asn Pro Lys Glu Ile Ile Leu Gly Glu Glu Arg Leu Glu Thr Gly Asn
            20                  25                  30

Thr Val Ala Asp Ile Ser Leu Gly Leu Ile Asn Phe Leu Tyr Ser Asn
        35                  40                  45

Phe Val Pro Gly Gly Gly Phe Ile Val Gly Leu Leu Glu Leu Ile Trp
    50                  55                  60

Gly Phe Ile Gly Pro Ser Gln Trp Asp Ile Phe Leu Ala Gln Ile Glu
65                  70                  75                  80

Gln Leu Ile Ser Gln Arg Ile Glu Glu Phe Ala Arg Asn Gln Ala Ile
                85                  90                  95

Ser Arg Leu Glu Gly Leu Ser Asn Leu Tyr Lys Val Tyr Val Arg Ala
            100                 105                 110

Phe Ser Asp Trp Glu Lys Asp Pro Thr Asn Pro Ala Leu Arg Glu Glu
        115                 120                 125

Met Arg Ile Gln Phe Asn Asp Met Asn Ser Ala Leu Ile Thr Ala Ile
    130                 135                 140

Pro Leu Phe Arg Val Gln Asn Tyr Glu Val Ala Leu Leu Ser Val Tyr
145                 150                 155                 160

Val Gln Ala Ala Asn Leu His Leu Ser Ile Leu Arg Asp Val Ser Val
                165                 170                 175

Phe Gly Glu Arg Trp Gly Tyr Asp Thr Ala Thr Ile Asn Asn Arg Tyr
            180                 185                 190

Ser Asp Leu Thr Ser Leu Ile His Val Tyr Thr Asn His Cys Val Asp
        195                 200                 205

Thr Tyr Asn Gln Gly Leu Arg Arg Leu Glu Gly Arg Phe Leu Ser Asp
    210                 215                 220

Trp Ile Val Tyr Asn Arg Phe Arg Arg Gln Leu Thr Ile Ser Val Leu
225                 230                 235                 240

Asp Ile Val Ala Phe Phe Pro Asn Tyr Asp Ile Arg Thr Tyr Pro Ile
                245                 250                 255

Gln Thr Ala Thr Gln Leu Thr Arg Glu Val Tyr Leu Asp Leu Pro Phe
            260                 265                 270

Ile Asn Glu Asn Leu Ser Pro Ala Ala Ser Tyr Pro Thr Phe Ser Ala

275                 280                 285

Ala Glu Ser Ala Ile Ile Arg Ser Pro His Leu Val Asp Phe Leu Asn
    290                 295                 300

Ser Phe Thr Ile Tyr Thr Asp Ser Leu Ala Arg Tyr Ala Tyr Trp Gly
305                 310                 315                 320

Gly His Leu Val Asn Ser Phe Arg Thr Gly Thr Thr Thr Asn Leu Ile
                325                 330                 335

Arg Ser Pro Leu Tyr Gly Arg Glu Gly Asn Thr Glu Arg Pro Val Thr
            340                 345                 350

Ile Thr Ala Ser Pro Ser Val Pro Ile Phe Arg Thr Leu Ser Tyr Ile
        355                 360                 365

Thr Gly Leu Asp Asn Ser Asn Pro Val Ala Gly Ile Glu Gly Val Glu
    370                 375                 380

Phe Gln Asn Thr Ile Ser Arg Ser Ile Tyr Arg Lys Ser Gly Pro Ile
385                 390                 395                 400

Asp Ser Phe Ser Glu Leu Pro Pro Gln Asp Ala Ser Val Ser Pro Ala
                405                 410                 415

Ile Gly Tyr Ser His Arg Leu Cys His Ala Thr Phe Leu Glu Arg Ile
            420                 425                 430

Ser Gly Pro Arg Ile Ala Gly Thr Val Phe Ser Trp Thr His Arg Ser
        435                 440                 445

Ala Ser Pro Thr Asn Glu Val Ser Pro Ser Arg Ile Thr Gln Ile Pro
    450                 455                 460

Trp Val Lys Ala His Thr Leu Ala Ser Gly Ala Ser Val Ile Lys Gly
465                 470                 475                 480

Pro Gly Phe Thr Gly Gly Asp Ile Leu Thr Arg Asn Ser Met Gly Glu
                485                 490                 495

Leu Gly Thr Leu Arg Val Thr Phe Thr Gly Arg Leu Pro Gln Ser Tyr
            500                 505                 510

Tyr Ile Arg Phe Arg Tyr Ala Ser Val Ala Asn Arg Ser Gly Thr Phe
        515                 520                 525

Arg Tyr Ser Gln Pro Pro Ser Tyr Gly Ile Ser Phe Pro Lys Thr Met
    530                 535                 540

Asp Ala Gly Glu Pro Leu Thr Ser Arg Ser Phe Ala His Thr Thr Leu
545                 550                 555                 560

Phe Thr Pro Ile Thr Phe Ser Arg Ala Gln Glu Glu Phe Asp Leu Tyr
                565                 570                 575

Ile Gln Ser Gly Val Tyr Ile Asp Arg Ile Glu Phe Ile Pro Val Thr
            580                 585                 590

Ala Thr Leu Glu Ala Glu Ser Asp Leu Glu Arg Ala Gln Lys Ala Val
        595                 600                 605

Asn Ala Leu Phe Thr Ser Ser Asn Gln Ile Gly Leu Lys Thr Asp Val
    610                 615                 620

Thr Asp Tyr His Ile Asp Arg Val Ser Asn Leu Val Glu Cys Leu Ser
625                 630                 635                 640

Asp Glu Phe Cys Leu Asp Glu Lys Lys Glu Leu Ser Glu Lys Val Lys
                645                 650                 655

His Ala Lys Arg Leu Ser Asp Glu Arg Asn Leu Leu Gln Asp Pro Asn
            660                 665                 670

Phe Arg Gly Ile Asn Arg Gln Leu Asp Arg Gly Trp Arg Gly Ser Thr
        675                 680                 685

Asp Ile Thr Ile Gln Gly Gly Asp Asp Val Phe Lys Glu Asn Tyr Val
    690                 695                 700

Thr Leu Leu Gly Thr Phe Asp Glu Cys Tyr Pro Thr Tyr Leu Tyr Gln
705                 710                 715                 720

Lys Ile Asp Glu Ser Lys Leu Lys Ala Tyr Thr Arg Tyr Gln Leu Arg
                725                 730                 735

Gly Tyr Ile Glu Asp Ser Gln Asp Leu Glu Ile Tyr Leu Ile Arg Tyr
            740                 745                 750

Asn Ala Lys His Glu Thr Val Asn Val Pro Gly Thr Gly Ser Leu Trp
        755                 760                 765

Pro Leu Ser Ala Pro Ser Pro Ile Gly Lys Cys Ala His His Ser His
    770                 775                 780

His Phe Ser Leu Asp Ile Asp Val Gly Cys Thr Asp Leu Asn Glu Asp
785                 790                 795                 800

Leu Gly Val Trp Val Ile Phe Lys Ile Lys Thr Gln Asp Gly His Ala
                805                 810                 815

Arg Leu Gly Asn Leu Glu Phe Leu Glu Glu Lys Pro Leu Val Gly Glu
            820                 825                 830

Ala Leu Ala Arg Val Lys Arg Ala Glu Lys Lys Trp Arg Asp Lys Arg
        835                 840                 845

Glu Lys Leu Glu Trp Glu Thr Asn Ile Val Tyr Lys Glu Ala Lys Glu
    850                 855                 860

Ser Val Asp Ala Leu Phe Val Asn Ser Gln Tyr Asp Arg Leu Gln Ala
865                 870                 875                 880

Asp Thr Asn Ile Ala Met Ile His Ala Ala Asp Lys Arg Val His Ser
                885                 890                 895

Ile Arg Glu Ala Tyr Leu Pro Glu Leu Ser Val Ile Pro Gly Val Asn
            900                 905                 910

Ala Ala Ile Phe Glu Glu Leu Glu Gly Arg Ile Phe Thr Ala Phe Ser
        915                 920                 925

Leu Tyr Asp Ala Arg Asn Val Ile Lys Asn Gly Asp Phe Asn Asn Gly
    930                 935                 940

Leu Ser Cys Trp Asn Val Lys Gly His Val Asp Val Glu Glu Gln Asn
945                 950                 955                 960

Asn His Arg Ser Val Leu Val Val Pro Glu Trp Glu Ala Glu Val Ser
                965                 970                 975

Gln Glu Val Arg Val Cys Pro Gly Arg Gly Tyr Ile Leu Arg Val Thr
            980                 985                 990

Ala Tyr Lys Glu Gly Tyr Gly Glu  Gly Cys Val Thr Ile  His Glu Ile
        995                 1000                 1005

Glu Asn  Asn Thr Asp Glu Leu  Lys Phe Ser Asn Cys  Val Glu Glu
    1010                 1015                 1020

Glu Val  Tyr Pro Asn Asn Thr  Val Thr Cys Asn Asp  Tyr Thr Ala
    1025                 1030                 1035

Thr Gln Glu Glu Tyr Glu Gly Thr Tyr Thr Ser Arg Asn Arg Gly
    1040                1045                1050

Tyr Asp Gly Ala Tyr Glu Ser Asn Ser Ser Val Pro Ala Asp Tyr
    1055                1060                1065

Ala Ser Ala Tyr Glu Glu Lys Ala Tyr Thr Asp Gly Arg Arg Asp
    1070                1075                1080

Asn Pro Cys Glu Ser Asn Arg Gly Tyr Gly Asp Tyr Thr Pro Leu
    1085                1090                1095

Pro Ala Gly Tyr Val Thr Lys Glu Leu Glu Tyr Phe Pro Glu Thr
    1100                1105                1110

Asp Lys Val Trp Ile Glu Ile Gly Glu Thr Glu Gly Thr Phe Ile
    1115                1120                1125

Val Asp Ser Val Glu Leu Leu Leu Met Glu Glu
    1130                1135

<210> 6
<211> 788
<212> PRT
<213> Bacillus thuringiensis

<220>
<221> VIP3Ab
<222> (1)..(788)

<400> 6

Met Ala Asn Met Asn Asn Thr Lys Leu Asn Ala Arg Ala Leu Pro Ser
1               5                   10                  15

Phe Ile Asp Tyr Phe Asn Gly Ile Tyr Gly Phe Ala Thr Gly Ile Lys
            20                  25                  30

Asp Ile Met Asn Met Ile Phe Lys Thr Asp Thr Gly Gly Asn Leu Thr
        35                  40                  45

Leu Asp Glu Ile Leu Lys Asn Gln Gln Leu Leu Asn Glu Ile Ser Gly
    50                  55                  60

Lys Leu Asp Gly Val Asn Gly Ser Leu Asn Asp Leu Ile Ala Gln Gly
65                  70                  75                  80

Asn Leu Asn Thr Glu Leu Ser Lys Glu Ile Leu Lys Ile Ala Asn Glu

85                  90                  95

Gln Asn Gln Val Leu Asn Asp Val Asn Asn Lys Leu Asp Ala Ile Asn
            100                 105                 110

Thr Met Leu His Ile Tyr Leu Pro Lys Ile Thr Ser Met Leu Ser Asp
        115                 120                 125

Val Met Lys Gln Asn Tyr Ala Leu Ser Leu Gln Val Glu Tyr Leu Ser
    130                 135                 140

Lys Gln Leu Lys Glu Ile Ser Asp Lys Leu Asp Val Ile Asn Val Asn
145                 150                 155                 160

Val Leu Ile Asn Ser Thr Leu Thr Glu Ile Thr Pro Ala Tyr Gln Arg
                165                 170                 175

Ile Lys Tyr Val Asn Glu Lys Phe Glu Glu Leu Thr Phe Ala Thr Glu
            180                 185                 190

Thr Thr Leu Lys Val Lys Lys Asp Ser Ser Pro Ala Asp Ile Leu Asp
        195                 200                 205

Glu Leu Thr Glu Leu Thr Glu Leu Ala Lys Ser Val Thr Lys Asn Asp
    210                 215                 220

Val Asp Gly Phe Glu Phe Tyr Leu Asn Thr Phe His Asp Val Met Val
225                 230                 235                 240

Gly Asn Asn Leu Phe Gly Arg Ser Ala Leu Lys Thr Ala Ser Glu Leu
                245                 250                 255

Ile Ala Lys Glu Asn Val Lys Thr Ser Gly Ser Glu Val Gly Asn Val
            260                 265                 270

Tyr Asn Phe Leu Ile Val Leu Thr Ala Leu Gln Ala Lys Ala Phe Leu
        275                 280                 285

Thr Leu Thr Thr Cys Arg Lys Leu Leu Gly Leu Ala Asp Ile Asp Tyr
    290                 295                 300

Thr Ser Ile Met Asn Glu His Leu Asn Lys Glu Lys Glu Glu Phe Arg
305                 310                 315                 320

Val Asn Ile Leu Pro Thr Leu Ser Asn Thr Phe Ser Asn Pro Asn Tyr
                325                 330                 335

Ala Lys Val Lys Gly Ser Asp Glu Asp Ala Lys Met Ile Val Glu Ala
            340                 345                 350

Lys Pro Gly His Ala Leu Val Gly Phe Glu Ile Ser Asn Asp Ser Met
        355                 360                 365

Thr Val Leu Lys Val Tyr Glu Ala Lys Leu Lys Gln Asn Tyr Gln Val
    370                 375                 380

Asp Lys Asp Ser Leu Ser Glu Val Ile Tyr Ser Asp Met Asp Lys Leu
385                 390                 395                 400

Leu Cys Pro Asp Gln Ser Glu Gln Ile Tyr Tyr Thr Asn Asn Ile Val
                405                 410                 415

Phe Pro Asn Glu Tyr Val Ile Thr Lys Ile Asp Phe Thr Lys Lys Met
            420                 425                 430

Lys Thr Leu Arg Tyr Glu Val Thr Ala Asn Ser Tyr Asp Ser Ser Thr
        435                 440                 445

Gly Glu Ile Asp Leu Asn Lys Lys Lys Val Glu Ser Ser Glu Ala Glu
    450                 455                 460

Tyr Arg Thr Leu Ser Ala Asn Asn Asp Gly Val Tyr Met Pro Leu Gly
465                 470                 475                 480

Val Ile Ser Glu Thr Phe Leu Thr Pro Ile Asn Gly Phe Gly Leu Gln
                485                 490                 495

Ala Asp Glu Asn Ser Arg Leu Ile Thr Leu Thr Cys Lys Ser Tyr Leu
            500                 505                 510

Arg Glu Leu Leu Leu Ala Thr Asp Leu Ser Asn Lys Glu Thr Lys Leu
        515                 520                 525

Ile Val Pro Pro Ile Ser Phe Ile Ser Asn Ile Val Glu Asn Gly Asn
    530                 535                 540

Leu Glu Gly Glu Asn Leu Glu Pro Trp Ile Ala Asn Asn Lys Asn Ala
545                 550                 555                 560

Tyr Val Asp His Thr Gly Gly Ile Asn Gly Thr Lys Val Leu Tyr Val
                565                 570                 575

His Lys Asp Gly Glu Phe Ser Gln Phe Val Gly Gly Lys Leu Lys Ser
            580                 585                 590

Lys Thr Glu Tyr Val Ile Gln Tyr Ile Val Lys Gly Lys Ala Ser Ile
        595                 600                 605

Tyr Leu Lys Asp Lys Lys Asn Glu Asn Ser Ile Tyr Glu Glu Ile Asn
    610                 615                 620

Asn Asp Leu Glu Gly Phe Gln Thr Val Thr Lys Arg Phe Ile Thr Gly
625                 630                 635                 640

Thr Asp Ser Ser Gly Ile His Leu Ile Phe Thr Ser Gln Asn Gly Glu
                645                 650                 655

Gly Ala Phe Gly Gly Asn Phe Ile Ile Ser Glu Ile Arg Thr Ser Glu
            660                 665                 670

Glu Leu Leu Ser Pro Glu Leu Ile Met Ser Asp Ala Trp Val Gly Ser
        675                 680                 685

Gln Gly Thr Trp Ile Ser Gly Asn Ser Leu Thr Ile Asn Ser Asn Val
    690                 695                 700

Asn Gly Thr Phe Arg Gln Asn Leu Pro Leu Glu Ser Tyr Ser Thr Tyr
705                 710                 715                 720

Ser Met Asn Phe Thr Val Asn Gly Phe Gly Lys Val Thr Val Arg Asn
                725                 730                 735

Ser Arg Glu Val Leu Phe Glu Lys Ser Tyr Pro Gln Leu Ser Pro Lys
            740                 745                 750

Asp Ile Ser Glu Lys Phe Thr Thr Ala Ala Asn Asn Thr Gly Leu Tyr
        755                 760                 765

Val Glu Leu Ser Arg Ser Thr Ser Gly Gly Ala Ile Asn Phe Arg Asp
    770                 775                 780

Phe Ser Ile Lys
785

<210> 7
<211> 612
<212> PRT
<213> Artificial

<220>
<223> Protease-Processed Cry1Ea Protein

<400> 7

Met Glu Ile Val Asn Asn Gln Asn Gln Cys Val Pro Tyr Asn Cys Leu
1               5                   10                  15

Asn Asn Pro Glu Asn Glu Ile Leu Asp Ile Glu Arg Ser Asn Ser Thr
            20                  25                  30

Val Ala Thr Asn Ile Ala Leu Glu Ile Ser Arg Leu Leu Ala Ser Ala
        35                  40                  45

Thr Pro Ile Gly Gly Ile Leu Leu Gly Leu Phe Asp Ala Ile Trp Gly
    50                  55                  60

Ser Ile Gly Pro Ser Gln Trp Asp Leu Phe Leu Glu Gln Ile Glu Leu
65                  70                  75                  80

Leu Ile Asp Gln Lys Ile Glu Glu Phe Ala Arg Asn Gln Ala Ile Ser
                85                  90                  95

Arg Leu Glu Gly Ile Ser Ser Leu Tyr Gly Ile Tyr Thr Glu Ala Phe
            100                 105                 110

Arg Glu Trp Glu Ala Asp Pro Thr Asn Pro Ala Leu Lys Glu Glu Met
        115                 120                 125

Arg Thr Gln Phe Asn Asp Met Asn Ser Ile Leu Val Thr Ala Ile Pro
    130                 135                 140

Leu Phe Ser Val Gln Asn Tyr Gln Val Pro Phe Leu Ser Val Tyr Val
145                 150                 155                 160

Gln Ala Ala Asn Leu His Leu Ser Val Leu Arg Asp Val Ser Val Phe
                165                 170                 175

Gly Gln Ala Trp Gly Phe Asp Ile Ala Thr Ile Asn Ser Arg Tyr Asn
            180                 185                 190

Asp Leu Thr Arg Leu Ile Pro Ile Tyr Thr Asp Tyr Ala Val Arg Trp
        195                 200                 205

Tyr Asn Thr Gly Leu Asp Arg Leu Pro Arg Thr Gly Gly Leu Arg Asn
    210                 215                 220

Trp Ala Arg Phe Asn Gln Phe Arg Arg Glu Leu Thr Ile Ser Val Leu
225                 230                 235                 240

Asp Ile Ile Ser Phe Phe Arg Asn Tyr Asp Ser Arg Leu Tyr Pro Ile
                245                 250                 255

Pro Thr Ser Ser Gln Leu Thr Arg Glu Val Tyr Thr Asp Pro Val Ile
        260                 265                 270

Asn Ile Thr Asp Tyr Arg Val Gly Pro Ser Phe Glu Asn Ile Glu Asn
        275                 280                 285

Ser Ala Ile Arg Ser Pro His Leu Met Asp Phe Leu Asn Asn Leu Thr
    290                 295                 300

Ile Asp Thr Asp Leu Ile Arg Gly Val His Tyr Trp Ala Gly His Arg
305                 310                 315                 320

Val Thr Ser His Phe Thr Gly Ser Ser Gln Val Ile Thr Thr Pro Gln
                325                 330                 335

Tyr Gly Ile Thr Ala Asn Ala Glu Pro Arg Arg Thr Ile Ala Pro Ser
        340                 345                 350

Thr Phe Pro Gly Leu Asn Leu Phe Tyr Arg Thr Leu Ser Asn Pro Phe
        355                 360                 365

Phe Arg Arg Ser Glu Asn Ile Thr Pro Thr Leu Gly Ile Asn Val Val
    370                 375                 380

Gln Gly Val Gly Phe Ile Gln Pro Asn Asn Ala Glu Val Leu Tyr Arg
385                 390                 395                 400

Ser Arg Gly Thr Val Asp Ser Leu Asn Glu Leu Pro Ile Asp Gly Glu
                405                 410                 415

Asn Ser Leu Val Gly Tyr Ser His Arg Leu Ser His Val Thr Leu Thr
        420                 425                 430

Arg Ser Leu Tyr Asn Thr Asn Ile Thr Ser Leu Pro Thr Phe Val Trp
        435                 440                 445

Thr His His Ser Ala Thr Asn Thr Asn Thr Ile Asn Pro Asp Ile Ile
    450                 455                 460

Thr Gln Ile Pro Leu Val Lys Gly Phe Arg Leu Gly Gly Gly Thr Ser
465                 470                 475                 480

Val Ile Lys Gly Pro Gly Phe Thr Gly Gly Asp Ile Leu Arg Arg Asn
                485                 490                 495

Thr Ile Gly Glu Phe Val Ser Leu Gln Val Asn Ile Asn Ser Pro Ile
        500                 505                 510

Thr Gln Arg Tyr Arg Leu Arg Phe Arg Tyr Ala Ser Ser Arg Asp Ala
        515                 520                 525

Arg Ile Thr Val Ala Ile Gly Gly Gln Ile Arg Val Asp Met Thr Leu
    530                 535                 540

Glu Lys Thr Met Glu Ile Gly Glu Ser Leu Thr Ser Arg Thr Phe Ser
545                 550                 555                 560

Tyr Thr Asn Phe Ser Asn Pro Phe Ser Phe Arg Ala Asn Pro Asp Ile
                565                 570                 575

Ile Arg Ile Ala Glu Glu Leu Pro Ile Arg Gly Gly Glu Leu Tyr Ile
            580                 585                 590

Asp Lys Ile Glu Leu Ile Leu Ala Asp Ala Thr Leu Glu Ala Glu Ser
        595                 600                 605

Asp Leu Glu Arg
    610

<210> 8
<211> 591
<212> PRT
<213> Artificial

<220>
<223> Protease-Processed Cry1Ab Protein

<400> 8

Ile Glu Thr Gly Tyr Thr Pro Ile Asp Ile Ser Leu Ser Leu Thr Gln
1               5                   10                  15

Phe Leu Leu Ser Glu Phe Val Pro Gly Ala Gly Phe Val Leu Gly Leu
            20                  25                  30

Val Asp Ile Ile Trp Gly Ile Phe Gly Pro Ser Gln Trp Asp Ala Phe
        35                  40                  45

Leu Val Gln Ile Glu Gln Leu Ile Asn Gln Arg Ile Glu Glu Phe Ala
    50                  55                  60

Arg Asn Gln Ala Ile Ser Arg Leu Glu Gly Leu Ser Asn Leu Tyr Gln
65                  70                  75                  80

Ile Tyr Ala Glu Ser Phe Arg Glu Trp Glu Ala Asp Pro Thr Asn Pro
                85                  90                  95

Ala Leu Arg Glu Glu Met Arg Ile Gln Phe Asn Asp Met Asn Ser Ala
            100                 105                 110

Leu Thr Thr Ala Ile Pro Leu Phe Ala Val Gln Asn Tyr Gln Val Pro
        115                 120                 125

Leu Leu Ser Val Tyr Val Gln Ala Ala Asn Leu His Leu Ser Val Leu
    130                 135                 140

Arg Asp Val Ser Val Phe Gly Gln Arg Trp Gly Phe Asp Ala Ala Thr
145                 150                 155                 160

Ile Asn Ser Arg Tyr Asn Asp Leu Thr Arg Leu Ile Gly Asn Tyr Thr
                165                 170                 175

Asp Tyr Ala Val Arg Trp Tyr Asn Thr Gly Leu Glu Arg Val Trp Gly
            180                 185                 190

Pro Asp Ser Arg Asp Trp Val Arg Tyr Asn Gln Phe Arg Arg Glu Leu
        195                 200                 205

Thr Leu Thr Val Leu Asp Ile Val Ala Leu Phe Pro Asn Tyr Asp Ser
    210                 215                 220

Arg Arg Tyr Pro Ile Arg Thr Val Ser Gln Leu Thr Arg Glu Ile Tyr
225                 230                 235                 240

Thr Asn Pro Val Leu Glu Asn Phe Asp Gly Ser Phe Arg Gly Ser Ala
                245                 250                 255

Gln Gly Ile Glu Arg Ser Ile Arg Ser Pro His Leu Met Asp Ile Leu
            260                 265                 270

Asn Ser Ile Thr Ile Tyr Thr Asp Ala His Arg Gly Tyr Tyr Tyr Trp
        275                 280                 285

Ser Gly His Gln Ile Met Ala Ser Pro Val Gly Phe Ser Gly Pro Glu
    290                 295                 300

Phe Thr Phe Pro Leu Tyr Gly Thr Met Gly Asn Ala Ala Pro Gln Gln
305                 310                 315                 320

Arg Ile Val Ala Gln Leu Gly Gln Gly Val Tyr Arg Thr Leu Ser Ser
                325                 330                 335

Thr Leu Tyr Arg Arg Pro Phe Asn Ile Gly Ile Asn Asn Gln Gln Leu
            340                 345                 350

Ser Val Leu Asp Gly Thr Glu Phe Ala Tyr Gly Thr Ser Ser Asn Leu
        355                 360                 365

Pro Ser Ala Val Tyr Arg Lys Ser Gly Thr Val Asp Ser Leu Asp Glu
    370                 375                 380

Ile Pro Pro Gln Asn Asn Asn Val Pro Pro Arg Gln Gly Phe Ser His
385                 390                 395                 400

Arg Leu Ser His Val Ser Met Phe Arg Ser Gly Phe Ser Asn Ser Ser
                405                 410                 415

Val Ser Ile Ile Arg Ala Pro Met Phe Ser Trp Ile His Arg Ser Ala
            420                 425                 430

Glu Phe Asn Asn Ile Ile Pro Ser Ser Gln Ile Thr Gln Ile Pro Leu
        435                 440                 445

Thr Lys Ser Thr Asn Leu Gly Ser Gly Thr Ser Val Val Lys Gly Pro
    450                 455                 460

Gly Phe Thr Gly Gly Asp Ile Leu Arg Arg Thr Ser Pro Gly Gln Ile
465                 470                 475                 480

Ser Thr Leu Arg Val Asn Ile Thr Ala Pro Leu Ser Gln Arg Tyr Arg
                485                 490                 495

Val Arg Ile Arg Tyr Ala Ser Thr Thr Asn Leu Gln Phe His Thr Ser
            500                 505                 510

Ile Asp Gly Arg Pro Ile Asn Gln Gly Asn Phe Ser Ala Thr Met Ser
        515                 520                 525

Ser Gly Ser Asn Leu Gln Ser Gly Ser Phe Arg Thr Val Gly Phe Thr
    530                 535                 540

Thr Pro Phe Asn Phe Ser Asn Gly Ser Ser Val Phe Thr Leu Ser Ala
545                 550                 555                 560

His Val Phe Asn Ser Gly Asn Glu Val Tyr Ile Asp Arg Ile Glu Phe
                565                 570                 575

Val Pro Ala Glu Val Thr Phe Glu Ala Glu Tyr Asp Leu Glu Arg
            580                 585                 590

<210> 9

<211>  616
<212>  PRT
<213>  Artificial

<220>
<223>  Truncated Cry1Be Protein

<400>  9

Ile Glu Asp Ser Leu Cys Ile Ala Glu Gly Asn Asn Ile Asp Pro Phe
1               5                   10                  15

Val Ser Ala Ser Thr Val Gln Thr Gly Ile Asn Ile Ala Gly Arg Ile
            20                  25                  30

Leu Gly Val Leu Gly Val Pro Phe Ala Gly Gln Ile Ala Ser Phe Tyr
        35                  40                  45

Ser Phe Leu Val Gly Glu Leu Trp Pro Arg Gly Arg Asp Pro Trp Glu
    50                  55                  60

Ile Phe Leu Glu His Val Glu Gln Leu Ile Arg Gln Gln Val Thr Glu
65                  70                  75                  80

Asn Thr Arg Asp Thr Ala Leu Ala Arg Leu Gln Gly Leu Gly Asn Ser
                85                  90                  95

Phe Arg Ala Tyr Gln Gln Ser Leu Glu Asp Trp Leu Glu Asn Arg Asp
            100                 105                 110

Asp Ala Arg Thr Arg Ser Val Leu Tyr Thr Gln Tyr Ile Ala Leu Glu
        115                 120                 125

Leu Asp Phe Leu Asn Ala Met Pro Leu Phe Ala Ile Arg Asn Gln Glu
    130                 135                 140

Val Pro Leu Leu Met Val Tyr Ala Gln Ala Ala Asn Leu His Leu Leu
145                 150                 155                 160

Leu Leu Arg Asp Ala Ser Leu Phe Gly Ser Glu Phe Gly Leu Thr Ser
                165                 170                 175

Gln Glu Ile Gln Arg Tyr Tyr Glu Arg Gln Val Glu Lys Thr Arg Glu
            180                 185                 190

Tyr Ser Asp Tyr Cys Ala Arg Trp Tyr Asn Thr Gly Leu Asn Asn Leu
        195                 200                 205

Arg Gly Thr Asn Ala Glu Ser Trp Leu Arg Tyr Asn Gln Phe Arg Arg
    210                 215                 220

Asp Leu Thr Leu Gly Val Leu Asp Leu Val Ala Leu Phe Pro Ser Tyr
225                 230                 235                 240

Asp Thr Arg Val Tyr Pro Met Asn Thr Ser Ala Gln Leu Thr Arg Glu
                245                 250                 255

Ile Tyr Thr Asp Pro Ile Gly Arg Thr Asn Ala Pro Ser Gly Phe Ala
            260                 265                 270

Ser Thr Asn Trp Phe Asn Asn Asn Ala Pro Ser Phe Ser Ala Ile Glu
        275                 280                 285

Ala Ala Val Ile Arg Pro Pro His Leu Leu Asp Phe Pro Glu Gln Leu
    290                 295                 300

Thr Ile Phe Ser Val Leu Ser Arg Trp Ser Asn Thr Gln Tyr Met Asn
305                 310                 315                 320

Tyr Trp Val Gly His Arg Leu Glu Ser Arg Thr Ile Arg Gly Ser Leu
                325                 330                 335

Ser Thr Ser Thr His Gly Asn Thr Asn Thr Ser Ile Asn Pro Val Thr
            340                 345                 350

Leu Gln Phe Thr Ser Arg Asp Val Tyr Arg Thr Glu Ser Phe Ala Gly
        355                 360                 365

Ile Asn Ile Leu Leu Thr Thr Pro Val Asn Gly Val Pro Trp Ala Arg
    370                 375                 380

Phe Asn Trp Arg Asn Pro Leu Asn Ser Leu Arg Gly Ser Leu Leu Tyr
385                 390                 395                 400

Thr Ile Gly Tyr Thr Gly Val Gly Thr Gln Leu Phe Asp Ser Glu Thr
                405                 410                 415

Glu Leu Pro Pro Glu Thr Thr Glu Arg Pro Asn Tyr Glu Ser Tyr Ser
            420                 425                 430

His Arg Leu Ser Asn Ile Arg Leu Ile Ser Gly Asn Thr Leu Arg Ala
        435                 440                 445

Pro Val Tyr Ser Trp Thr His Arg Ser Ala Asp Arg Thr Asn Thr Ile
    450                 455                 460

Ser Ser Asp Ser Ile Thr Gln Ile Pro Leu Val Lys Ser Phe Asn Leu

465                 470                 475                 480

Asn Ser Gly Thr Ser Val Val Ser Gly Pro Gly Phe Thr Gly Gly Asp
                485                 490                 495

Ile Ile Arg Thr Asn Val Asn Gly Ser Val Leu Ser Met Gly Leu Asn
            500                 505                 510

Phe Asn Asn Thr Ser Leu Gln Arg Tyr Arg Val Arg Val Arg Tyr Ala
        515                 520                 525

Ala Ser Gln Thr Met Val Leu Arg Val Thr Val Gly Gly Ser Thr Thr
    530                 535                 540

Phe Asp Gln Gly Phe Pro Ser Thr Met Ser Ala Asn Glu Ser Leu Thr
545                 550                 555                 560

Ser Gln Ser Phe Arg Phe Ala Glu Phe Pro Val Gly Ile Ser Ala Ser
                565                 570                 575

Gly Ser Gln Thr Ala Gly Ile Ser Ile Ser Asn Asn Ala Gly Arg Gln
            580                 585                 590

Thr Phe His Phe Asp Lys Ile Glu Phe Ile Pro Ile Thr Ala Thr Leu
        595                 600                 605

Glu Ala Glu Ser Asp Leu Glu Arg
    610                 615

<210>  10
<211>  600
<212>  PRT
<213>  Artificial

<220>
<223>  Truncated Cry1Ca Protein

<400>  10

Ile Ser Thr Gly Asn Ser Ser Ile Asp Ile Ser Leu Ser Leu Val Gln
1               5                   10                  15

Phe Leu Val Ser Asn Phe Val Pro Gly Gly Gly Phe Leu Val Gly Leu
            20                  25                  30

Ile Asp Phe Val Trp Gly Ile Val Gly Pro Ser Gln Trp Asp Ala Phe
        35                  40                  45

Leu Val Gln Ile Glu Gln Leu Ile Asn Glu Arg Ile Ala Glu Phe Ala
    50                  55                  60

Arg Asn Ala Ala Ile Ala Asn Leu Glu Gly Leu Gly Asn Asn Phe Asn
65                  70                  75                  80

Ile Tyr Val Glu Ala Phe Lys Glu Trp Glu Glu Asp Pro Lys Asn Pro
                85                  90                  95

Ala Thr Arg Thr Arg Val Ile Asp Arg Phe Arg Ile Leu Asp Gly Leu
            100                 105                 110

Leu Glu Arg Asp Ile Pro Ser Phe Arg Ile Ser Gly Phe Glu Val Pro
        115                 120                 125

Leu Leu Ser Val Tyr Ala Gln Ala Ala Asn Leu His Leu Ala Ile Leu
    130                 135                 140

Arg Asp Ser Val Ile Phe Gly Glu Arg Trp Gly Leu Thr Thr Ile Asn
145                 150                 155                 160

Val Asn Glu Asn Tyr Asn Arg Leu Ile Arg His Ile Asp Glu Tyr Ala
                165                 170                 175

Asp His Cys Ala Asn Thr Tyr Asn Arg Gly Leu Asn Asn Leu Pro Lys
            180                 185                 190

Ser Thr Tyr Gln Asp Trp Ile Thr Tyr Asn Arg Leu Arg Arg Asp Leu
        195                 200                 205

Thr Leu Thr Val Leu Asp Ile Ala Ala Phe Phe Pro Asn Tyr Asp Asn
    210                 215                 220

Arg Arg Tyr Pro Ile Gln Pro Val Gly Gln Leu Thr Arg Glu Val Tyr
225                 230                 235                 240

Thr Asp Pro Leu Ile Asn Phe Asn Pro Gln Leu Gln Ser Val Ala Gln
                245                 250                 255

Leu Pro Thr Phe Asn Val Met Glu Asn Ser Ala Ile Arg Asn Pro His
            260                 265                 270

Leu Phe Asp Ile Leu Asn Asn Leu Thr Ile Phe Thr Asp Trp Phe Ser
        275                 280                 285

Val Gly Arg Asn Phe Tyr Trp Gly Gly His Arg Val Ile Ser Ser Leu
    290                 295                 300

Ile Gly Gly Gly Asn Ile Thr Ser Pro Ile Tyr Gly Arg Glu Ala Asn

305                 310                 315                 320

Gln Glu Pro Pro Arg Ser Phe Thr Phe Asn Gly Pro Val Phe Arg Thr
                325                 330                 335

Leu Ser Asn Pro Thr Leu Arg Leu Leu Gln Gln Pro Trp Pro Ala Pro
            340                 345                 350

Pro Phe Asn Leu Arg Gly Val Glu Gly Val Glu Phe Ser Thr Pro Thr
        355                 360                 365

Asn Ser Phe Thr Tyr Arg Gly Arg Gly Thr Val Asp Ser Leu Thr Glu
    370                 375                 380

Leu Pro Pro Glu Asp Asn Ser Val Pro Pro Arg Glu Gly Tyr Ser His
385                 390                 395                 400

Arg Leu Cys His Ala Thr Phe Val Gln Arg Ser Gly Thr Pro Phe Leu
                405                 410                 415

Thr Thr Gly Val Val Phe Ser Trp Thr His Arg Ser Ala Thr Leu Thr
            420                 425                 430

Asn Thr Ile Asp Pro Glu Arg Ile Asn Gln Ile Pro Leu Val Lys Gly
        435                 440                 445

Phe Arg Val Trp Gly Gly Thr Ser Val Ile Thr Gly Pro Gly Phe Thr
    450                 455                 460

Gly Gly Asp Ile Leu Arg Arg Asn Thr Phe Gly Asp Phe Val Ser Leu
465                 470                 475                 480

Gln Val Asn Ile Asn Ser Pro Ile Thr Gln Arg Tyr Arg Leu Arg Phe
                485                 490                 495

Arg Tyr Ala Ser Ser Arg Asp Ala Arg Val Ile Val Leu Thr Gly Ala
            500                 505                 510

Ala Ser Thr Gly Val Gly Gly Gln Val Ser Val Asn Met Pro Leu Gln
        515                 520                 525

Lys Thr Met Glu Ile Gly Glu Asn Leu Thr Ser Arg Thr Phe Arg Tyr
    530                 535                 540

Thr Asp Phe Ser Asn Pro Phe Ser Phe Arg Ala Asn Pro Asp Ile Ile
545                 550                 555                 560

Gly Ile Ser Glu Gln Pro Leu Phe Gly Ala Gly Ser Ile Ser Ser Gly
                565                 570                 575

Glu Leu Tyr Ile Asp Lys Ile Glu Ile Ile Leu Ala Asp Ala Thr Leu
            580                 585                 590

Glu Ala Glu Ser Asp Leu Glu Arg
        595                 600

<210>  11
<211>  576
<212>  PRT
<213>  Artificial

<220>
<223>  Truncated Cry1Da Protein

<400>  11

Leu Glu Thr Gly Asn Thr Val Ala Asp Ile Ser Leu Gly Leu Ile Asn
1               5                   10                  15

Phe Leu Tyr Ser Asn Phe Val Pro Gly Gly Gly Phe Ile Val Gly Leu
            20                  25                  30

Leu Glu Leu Ile Trp Gly Phe Ile Gly Pro Ser Gln Trp Asp Ile Phe
        35                  40                  45

Leu Ala Gln Ile Glu Gln Leu Ile Ser Gln Arg Ile Glu Glu Phe Ala
    50                  55                  60

Arg Asn Gln Ala Ile Ser Arg Leu Glu Gly Leu Ser Asn Leu Tyr Lys
65                  70                  75                  80

Val Tyr Val Arg Ala Phe Ser Asp Trp Glu Lys Asp Pro Thr Asn Pro
                85                  90                  95

Ala Leu Arg Glu Glu Met Arg Ile Gln Phe Asn Asp Met Asn Ser Ala
            100                 105                 110

Leu Ile Thr Ala Ile Pro Leu Phe Arg Val Gln Asn Tyr Glu Val Ala
        115                 120                 125

Leu Leu Ser Val Tyr Val Gln Ala Ala Asn Leu His Leu Ser Ile Leu
    130                 135                 140

Arg Asp Val Ser Val Phe Gly Glu Arg Trp Gly Tyr Asp Thr Ala Thr
145                 150                 155                 160

Ile Asn Asn Arg Tyr Ser Asp Leu Thr Ser Leu Ile His Val Tyr Thr

                165                 170                 175

Asn His Cys Val Asp Thr Tyr Asn Gln Gly Leu Arg Arg Leu Glu Gly
            180                 185                 190

Arg Phe Leu Ser Asp Trp Ile Val Tyr Asn Arg Phe Arg Arg Gln Leu
        195                 200                 205

Thr Ile Ser Val Leu Asp Ile Val Ala Phe Phe Pro Asn Tyr Asp Ile
    210                 215                 220

Arg Thr Tyr Pro Ile Gln Thr Ala Thr Gln Leu Thr Arg Glu Val Tyr
225                 230                 235                 240

Leu Asp Leu Pro Phe Ile Asn Glu Asn Leu Ser Pro Ala Ala Ser Tyr
                245                 250                 255

Pro Thr Phe Ser Ala Ala Glu Ser Ala Ile Ile Arg Ser Pro His Leu
            260                 265                 270

Val Asp Phe Leu Asn Ser Phe Thr Ile Tyr Thr Asp Ser Leu Ala Arg
        275                 280                 285

Tyr Ala Tyr Trp Gly Gly His Leu Val Asn Ser Phe Arg Thr Gly Thr
    290                 295                 300

Thr Thr Asn Leu Ile Arg Ser Pro Leu Tyr Gly Arg Glu Gly Asn Thr
305                 310                 315                 320

Glu Arg Pro Val Thr Ile Thr Ala Ser Pro Ser Val Pro Ile Phe Arg
                325                 330                 335

Thr Leu Ser Tyr Ile Thr Gly Leu Asp Asn Ser Asn Pro Val Ala Gly
            340                 345                 350

Ile Glu Gly Val Glu Phe Gln Asn Thr Ile Ser Arg Ser Ile Tyr Arg
        355                 360                 365

Lys Ser Gly Pro Ile Asp Ser Phe Ser Glu Leu Pro Pro Gln Asp Ala
    370                 375                 380

Ser Val Ser Pro Ala Ile Gly Tyr Ser His Arg Leu Cys His Ala Thr
385                 390                 395                 400

Phe Leu Glu Arg Ile Ser Gly Pro Arg Ile Ala Gly Thr Val Phe Ser
                405                 410                 415

Trp Thr His Arg Ser Ala Ser Pro Thr Asn Glu Val Ser Pro Ser Arg
                420                 425                 430

Ile Thr Gln Ile Pro Trp Val Lys Ala His Thr Leu Ala Ser Gly Ala
            435                 440                 445

Ser Val Ile Lys Gly Pro Gly Phe Thr Gly Gly Asp Ile Leu Thr Arg
        450                 455                 460

Asn Ser Met Gly Glu Leu Gly Thr Leu Arg Val Thr Phe Thr Gly Arg
465                 470                 475                 480

Leu Pro Gln Ser Tyr Tyr Ile Arg Phe Arg Tyr Ala Ser Val Ala Asn
                485                 490                 495

Arg Ser Gly Thr Phe Arg Tyr Ser Gln Pro Pro Ser Tyr Gly Ile Ser
                500                 505                 510

Phe Pro Lys Thr Met Asp Ala Gly Glu Pro Leu Thr Ser Arg Ser Phe
            515                 520                 525

Ala His Thr Thr Leu Phe Thr Pro Ile Thr Phe Ser Arg Ala Gln Glu
        530                 535                 540

Glu Phe Asp Leu Tyr Ile Gln Ser Gly Val Tyr Ile Asp Arg Ile Glu
545                 550                 555                 560

Phe Ile Pro Val Thr Ala Thr Leu Glu Ala Glu Ser Asp Leu Glu Arg
                565                 570                 575